# EUROPEAN PATENT SPECIFICATION

(11) **EP 1 490 407 B1**
(45) Date of publication and mention of the grant of the patent: **16.05.2007**
(21) Application number: 03745713.2
(22) Date of filing: 04.04.2003
(51) Int. Cl.: C07K 16/28, A61K 39/395, C07K 14/025, A61K 39/12

(54) **INDUCTION OF ANTI-TUMOR CTL IMMUNITY THROUGH IN VIVO TRIGGERING OF 4-1BB AND/OR CD40**
INDUKTION DER ANTI-TUMOR-CTL-IMMUNITÄT DURCH IN-VIVO-AKTIVIERUNG VON 4-1BB UND/ODER CD40
INDUCTION DE L'IMMUNITE ANTITUMORALE CTL PAR UN DECLENCHEMENT IN VIVO DE 4-1BB ET/OU CD40

(30) Priority: 04.04.2002 US 115620
(43) Date of publication of application: 29.12.2004
(73) Proprietor: Academisch Ziekenhuis Leiden h.o.d.n. Leids Universitair Medisch Centrum, 2333 ZA Leiden (NL)
(72) Inventor: DIEHL, Linda, NL-2351 EG Leiderdorp (NL); VAN MIERLO, Geertje, NL-2341 JZ Oegstgeest (NL); MELIEF, Cornelis, J., M., NL-2012 KC Haarlem (NL); TOES, Rene, NL-2311 HN Leiden (NL); OFFRINGA, Rienk, NL-2313 SJ Leiden (NL)
(74) Representative: van Westenbrugge, Andries
(86) International application number: PCT/NL2003/000254
(87) International publication number: WO 2003/084999

(56) References cited:
- WO-A-99/61065
- MELIEF C J M ET AL: "STRATEGIES FOR IMMUNOTHERAPY OF CANCER" ADVANCES IN IMMUNOLOGY, ACADEMIC PRESS INC., NEW YORK, NY, US, vol. 75, 2000, pages 235-282, XP001027072 ISSN: 0065-2776
- WILCOX R A ET AL: "Provision of antigen and CD137 signaling breaks immunological ignorance, promoting regression of poorly immunogenic tumors" JOURNAL OF CLINICAL INVESTIGATION, NEW YORK, NY, US, vol. 109, no. 5, March 2002 (2002-03), pages 651-659, XP002238985 ISSN: 0021-9738
- MELERO I ET AL: "Monoclonal antibodies against the 4-1BB T-cell activation molecule eradicate established tumors" NATURE MEDICINE, NATURE PUBLISHING, CO, US, vol. 3, no. 6, June 1997 (1997-06), pages 682-685, XP002104261 ISSN: 1078-8956 cited in the application

## Description

### Field of the invention

The present invention relates to methods and compositions for treating tumors or infectious diseases, wherein the composition comprises an agonistic anti-CD40 antibody or a fragment thereof. The compositions may further comprise polypeptides or nucleic acid sequences having or encoding a class I MHC or class II MHC-restricted T cell epitope.

### Background of the invention

Many tumors escape surveillance by our immune system. In cancer patients there is clearly a quantitative and/or qualitative defect in the immune system's specific mechanisms to delete tumor cells. One of these mechanisms is provided by the cytotoxic T cells (CTL) that can recognise and kill cells infected by virus or transformed into cancer cells. Previously it was postulated that dendritic cells (DC) stimulate T-helper cells which, in turn, provide help for the activation of CTL. The present inventors have demonstrated that the T-helper cell is not providing helper signals directly to the CTL (by secretion of IL2), but rather, the T-helper cell is providing a signal to the DC that induces yet uncharacterised cell surface and/or soluble molecules that can activate CTL in the absence of T-helper cells. The signal provided by the T-helper cell to the DC is mediated by CD40L-CD40 interaction. This novel finding has provided an unique opportunity for cancer immunotherapy.

The immune system is capable of killing autologous cells when they become infected by virus or when they transform into cancer cells. Such a potentially dangerous mechanism must, clearly, be under tight control. The immune system's CTL circulate as inactive precursors. To be activated, the precursor T-killer cell must recognise its specific antigen peptide, which is presented as MHC class I molecules on professional APC. However, in order to prime these T cells, the APC also need to present the antigen in a proper costimulatory context as provided by, amongst others, the costimulatory surface molecules B7.1 and B7.2 and by the lympokine IL-12.

Until recently it was believed that a T-helper cell that recognises the same antigen on the same APC is needed to fully activate the CTL. The specific T-helper cell would supply cytokines such as IL-2 needed for the activation of the CTL. Guerder and Matzinger (J. Exp. Med. 176:553 (1992)), however, proposed the "licensing" model for CTL activation. In this model it was suggested that the T-helper cell, when recognising its antigen on a professional APC, would deliver an activation signal to the APC that as a result would be able to subsequently activate a CTL without the need for the T-helper cell to be present. Only very recently, the molecular mechanism of the licensing model has been elucidated. Schoenberger et al. (Nature 393:480 (1998)), described the role of the CD40L-CD40 pathway in the licensing model. Interaction between T-helper cell and DC through the CD40-CD40L binding results in activation of the DC, thereby enabling the DC to efficiently prime naive CTL.

DC circulate through the tissues of our body and in this manner can collect, process and present antigens. After collection of antigens, they migrate to the draining lymph nodes where they present antigen to the T cells. It is well known that a DC needs to be activated to perform optimally. Resting DC express only modest levels of MHC and costimulatory molecules and are poor stimulators of T cells. DC can be activated by inflammatory cytokines and bacterial products, which results in upregulation of MHC and costimulatory molecules. Activation of DC into fully mature DC, expressing optimal levels of MHC molecules, costimulatory molecules and lymphokines such as IL-12, requires additional triggering of these cells through the CD40 receptor. Consequently, the combination of inflammatory cytokines at the site of antigen uptake and the CD40L-CD40 interaction during the T-helper cell interaction result in an optimal capacity to license the DC for CTL activation.

Many tumors escape immune surveillance by specific CTL mechanisms. If DC gather tumor antigens under non-inflammatory conditions the number of T-helper cells that are activated may be to low to induce enough CTL to be activated to induce an appropriate anti-tumor response. This concept has prompted investigators to help the immune system by administration of cytokines such as IL-2 and IL-12 that directly stimulate CTL activity or by boosting antigen presentation by administration of tumor cells transfected with GM-CSF. These strategies have met variable but encouraging results.

It is clear that there is still a great need to find ways to generate and/or enhance protective anti-tumor responses involving cellular and humoral immunity. The CD40 activation pathway was found to be a major immunoregulatory pathway for the generation of primary humoral and cellular immune responses. As described above, the CD40 pathway on DC is responsible for the induction of anti-tumor CTL responses. In addition, activation of the CD40 pathway on macrophages stimulates a strong tumoricidal activity.

The cardinal effect of CD40-triggering, either by CD40L⁺ Th cells or by agonistic anti-CD40 Ab (in case sufficient T cell help is not available), is the maturation of DC, which endows these cells with the capacity to present antigens in the context of the costimulatory signals required for priming of naive CTL (5). The costimulatory signal delivered by CD80 and CD86, which are both expressed at high levels on mature DC, to the CD28 receptor on T cells has been investigated most extensively (reviewed in: (6, 7)). Although this signal plays a pivotal role in T cell activation, a number of additional costimulatory pathways also contribute in this process. One of these involves the 4-1 BB/4-1 BBL receptor-ligand pair. 4-1 BB is a member of the TNF-receptor (TNFR) family and is expressed on activated CD8⁺ and CD4⁺ T cells (8, 9). Its natural ligand, 4-1BBL, is expressed on B cells, macrophages and DC (10-12). In vitro studies have shown that stimulation of T cells with agonistic anti-4-1BB Ab increased TCR-induced proliferation and cytokine production by T cells. Although both CD4⁺ Th cells and CD8⁺ CTL could be costimulated in this way, CD8⁺ T cells were more responsive to 4-1BB triggering than CD4⁺ T cells (8, 13). Accordingly, blocking of 4-1BB costimulation in vitro was shown to inhibit APC-mediated T cell activation (14, 15), and 4-1BBL-deficient mice showed a reduced capacity to raise CTL immunity against viral infections (16-18). Conversely, administration of agonistic anti-4-1BB Ab in mice was shown to amplify the generation of CTL in a murine model for graft versus host disease (13), and also resulted in the rejection of poorly immunogenic tumors in tumor-bearing mice (19).

### Summary of the Invention

The invention relates to CD40 binding molecules, that may be used as such or together with CTL-activating peptides, including tumor antigens or antigens of infectious agents, in a pharmaceutical composition. Such composition is useful for enhancing the anti-tumor effect of a peptide tumor vaccine, or for otherwise activating CTLs so that the activated CTLs can act against tumorous or infected cells. The CD40 binding molecules can include antibody molecules, as well as homologues, analogues and modified or derived forms thereof, including immunoglobulin fragments like Fab, (Fab')₂ and Fv, as well as small molecules including peptides, oligonucleotides, peptidomimetics and organic compounds which bind to CD40 and activate the CTL response. CTL-activating peptides include the adenovirus-derived E1A peptide, having the sequence SGPSNTPPEI (SEQ ID NO:2), and the HPV 16 E7 peptide derived from human papillomavirus type 16, having the sequence RAHYNIVTF (SEQ ID NO:3).

The CD40 binding molecule, and the CTL activating peptide can be administered to a patient by suitable means, including injection, or gene constructs encoding such a molecule and a peptide can be administered, and the molecule and peptide thereby produced *in vivo* or *ex vivo.* Such a gene therapy is conducted according to methods well known in the art. If the transfection or infection of the gene constructs is done *ex vivo,* the infected or transfected cells can be administered to the patient, or these steps can be done *in vivo* whereby the molecule and the peptide are produced endogenously. The transfection or infection, if done *ex vivo,* can be by conventional methods, including electroporation, calcium phosphate transfection, micro-injection or by incorporating the gene constructs into suitable liposomes. Vectors, including a retrovirus, adenovirus or a parvovirus vector, or plasmids, can be used to incorporate the gene constructs, which are then expressed *in vivo* or *ex vivo.*

It is demonstrated herein that T-cell help for CTL priming is mediated through CD40-CD40Ligand (CD40L) interactions, and that lack of CTL priming in the absence of CD4⁺ T cells can be restored by monoclonal antibody (mAb)-mediated CD40 activation of bone marrow-derived APC in the presence of CTL-activating peptides including tumor antigens. Furthermore, blockade of CD40L, expressed by CD4⁺ T cells, results in the failure to raise CTL immunity. This defect can be overcome by *in vivo* CD40-triggering. *In vivo* triggering of CD40 can markedly enhance the efficacy of peptide-based anti-tumor vaccines, or otherwise activate CTLs to result in an anti-tumor or anti-infected cell reaction.

It is also noted that a CTL-activating peptide can become tolerogenic, meaning that the host reaction against cells expressing such peptide is inhibited, in the absence of anti-CD40. However, such a peptide combined with an activating anti-CD40 antibody converts tolerization into strong CTL activation. Moreover, as noted above, CD40-ligation can provide an already protective tumor-specific peptide-vaccine with the capacity to induce therapeutic CTL immunity in tumor-bearing mice.

These findings together demonstrate that the CD40-CD40Ligand pair acts as a switch determining whether naive peripheral CTL are primed or tolerized, Therefore CD40-binding agents such as monoclonal antibodies and other stimulatory ligands can be effectively used in combination with a CTL-activating peptide.

It is further demonstrated herein that triggering of 4-1BB through e.g. agonistic anti-4-1BB antibodies delivers a powerful costimulatory signal to CTL, which signal can effectively replace the need for CD4⁺ T cell help in cross-priming of antigen-specific CTL immunity. 4-1BB can convert an otherwise tolerogenic peptide vaccine into a formulation capable of efficient CTL priming. Initial activation of naive CTL can occur in the absence of 4-1BB costimulation but this signal permits increased survival of antigen-stimulated CTL. The triggering of T-helper-independent CTL immunity by 4-1BB costimulation does require prior stimulation of the TCR and CD-28 dependent costimulation. The 4-1BB-mediated survival-signal is thus positioned downstream of antigen-specific TCR triggering and CD-28-dependent costimulation of naive CTL. 4-1BB binding molecules, as such or in combination with CD40 binding molecule and/or CTL-activating peptides, may thus be used for the preparation of medicaments for the induction, amplification and persistence of antigen-specific CTL responses, e.g. in increasing the potency of anti-tumor vaccines.

### Brief description of the Figures

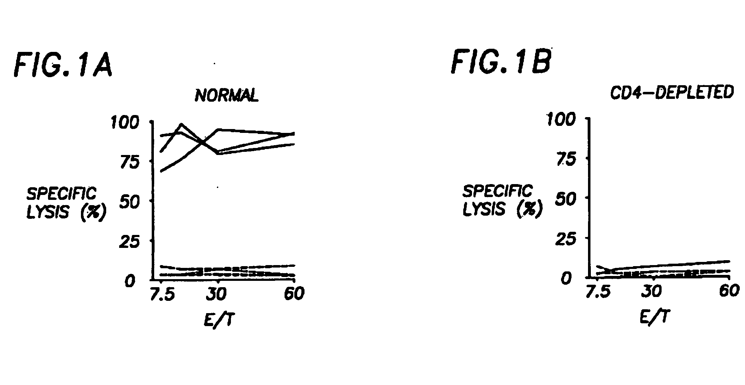

### Figure 1: Cross-priming of E1B-specific CTLs requires CD4⁺ T helper cells

Splenocytes from normal (**a**) or CD4-depleted B6 (**b**) mice immunized with Ad5E1-BALB/c MECs were tested at various effector/target ratios for lysis of syngeneic MEC target cells loaded with the E1B₁₉₂₋₂₀₀ peptide (solid lines), which is derived from human adenovirus and has the sequence VNIRNCCYI (SEQ ID NO: 1)or a D^{d}-restricted control peptide HPV-16E7₄₉₋₅₇ (dashed lines). Each line represents one mouse. Data shown represent one experiment of three performed with similar results.

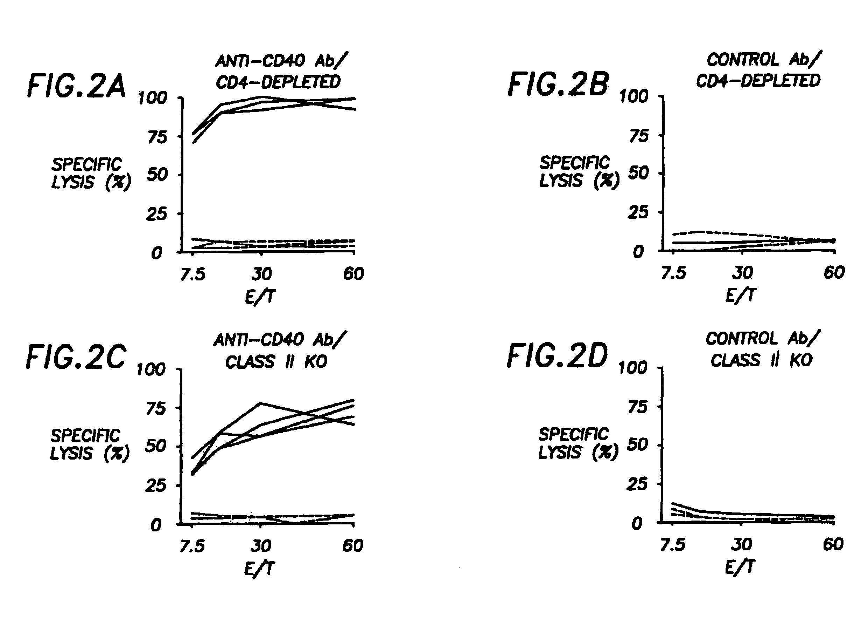

### Figure 2: CD40 activation replaces CD4⁺ T helper cells

Splenocytes from CD4-depleted (**a, b**) or classII-deficient I-Ab-knockout (KO) (c,d) B6 mice were immunized with Ad5E1-BALB/c MECs and treated with either the CD40-activating antibody (Ab) FGK45 (**a, c**) or an isotype control antibody (**b, d**). These splenocytes were tested for E1B-specific CTL activity on syngeneic MEC target cells loaded with either the E1B₁₉₂₋₂₀₀ peptide (solid lines) or the HPV-16 E7₄₉₋₅₇ control peptide (dashed lines). Each line represents a single mouse. Data depicted are from two independent experiments. E/T ratio, effector/target ratio.

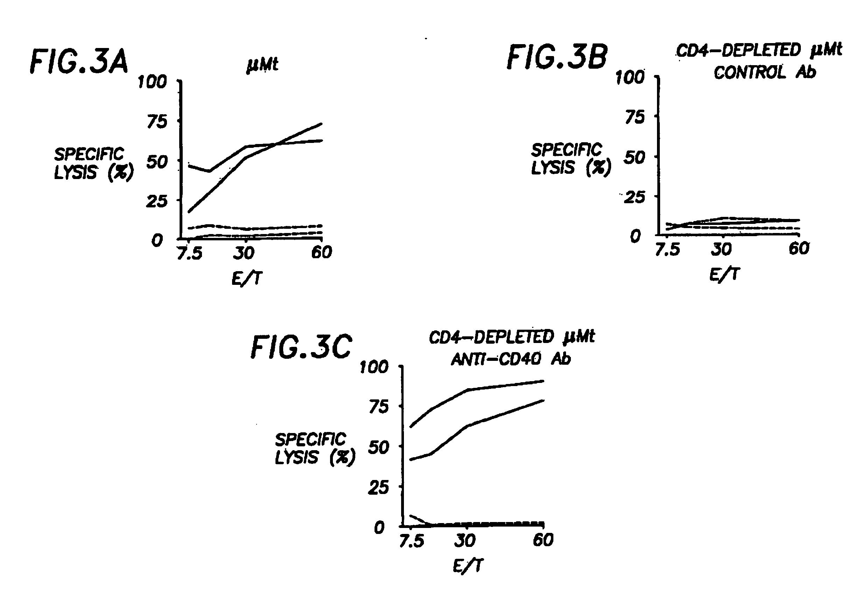

### Figure 3: B cells are not essential as cross-priming APCs or for anti-CD40-mediated restoration of cross-priming

Splenocytes were taken from untreated (**a**), CD4-depleted B-cell-deficient B6 MT mice (**b, c**), which were immunized with Ad5E1-BALB/c MECs and which received either an isotype control antibody (**b**) or the CD40-activating antibody FGK45 (**c**). These splenocytes were tested for E1B-specific CTL activity on syngeneic MEC target cells loaded with either the E1B₁₉₂₋₂₀₀ peptide (solid lines) or the HPV E7₄₉₋₅₇ control peptide (dashed lines). Each line represents one mouse. Data shown represent one experiment of two performed with similar results.

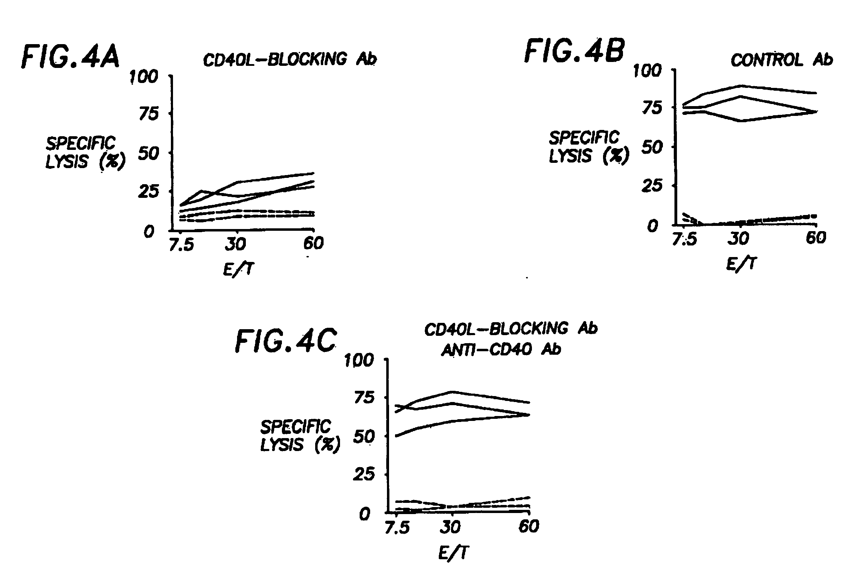

### Figure 4: CD40L blockade prevents cross-priming of E1B-specific CTLs

Splenocytes were taken from B6 mice immunized with Ad5E1-BALB/c MECs and treated with the CD40L-blocking antibody MR-1 (**a**), or control antibody (**b**), or from mice treated with the CD40L-blocking antibody MR-1 in combination with the CD40-activating antibody FGK45 (c) 24h after immunization. These splenocytes were tested for E1B-specific CTL activity on syngeneic MEC target cells loaded with the E1B₁₉₂₋₂₀₀ peptide (solid lines) or the HPV-16 E7₄₉₋₅₇ control peptide (dashed lines). Each line represents one mouse. Data shown represent one experiment of three performed with similar results. E/T ratio, effector/target ratio.

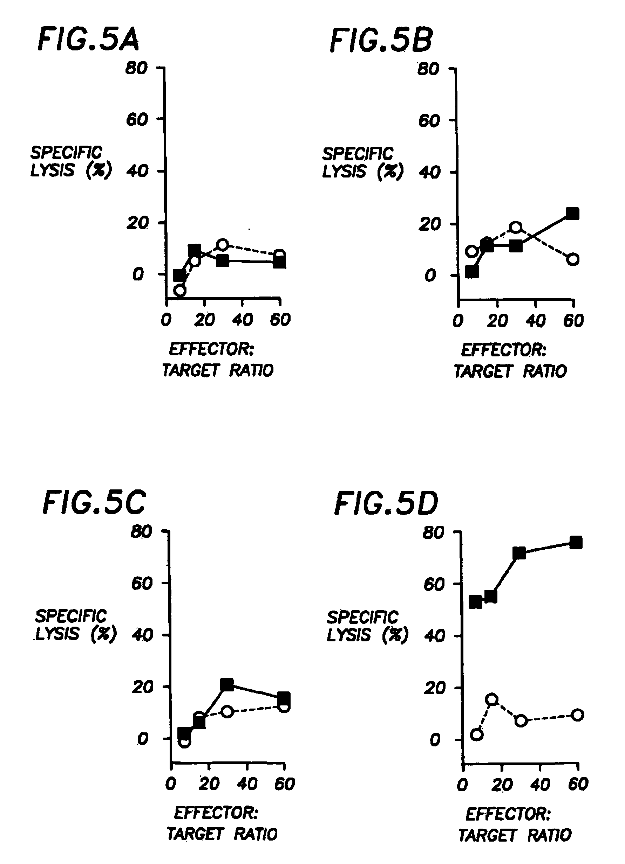

### Figure 5: Mice injected s.c. with the E1A-peptide are no longer able to mount E1A-specific CTL

C57BL/6 mice were injected twice s.c. (1 week interval) with 20 µg E1A-peptide (**a, b**) or control-peptide (**c, d**) in IFA, and challenged i.p. 1 day later with SAMB7 (**b**, **d**), a cell line expressing high amounts of E1A-peptide. Bulk cultures derived from these mice were tested for E1A-specific cytotoxicity on target cells pulsed with the E1A-peptide (-■-) or the HPV16 E7-peptide (-O-). Specific lysis of representative bulk cultures at different effector to target (E/T) ratios is shown. This experiment has been repeated 4 times with comparable results.

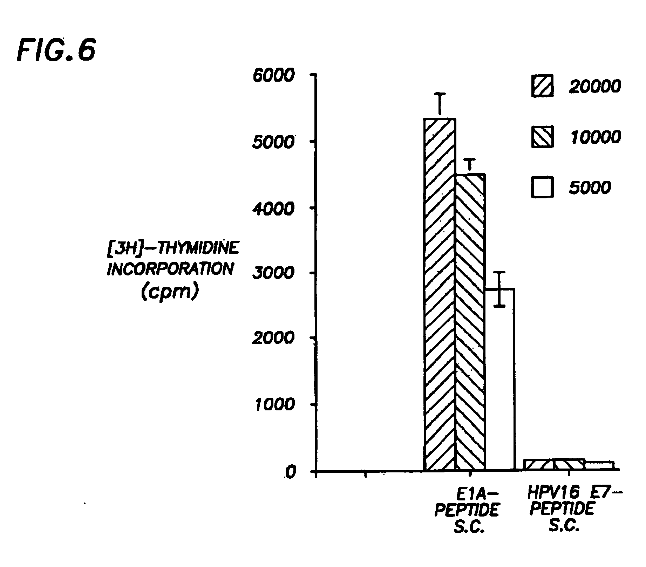

### Figure 6: Tolerizing E1A-peptide is rapidly distributed systemically after s.c. injection in IFA

Spleen cells derived from untreated C57BL/6 mice (-), or from mice injected s.c. 16 h earlier with 100 µg of E1A- or HPV16 E7-peptide in IFA were used as stimulator cells for an E1A-specific CTL clone. [³H]-thymidine incorporation (cpm) +/- S.E.M. is shown for different effector to stimulator concentrations, without subtraction of background counts. Results are representative of 5 independent experiments.

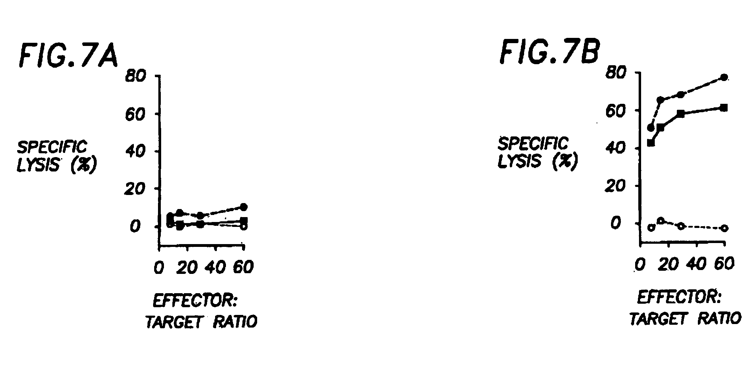

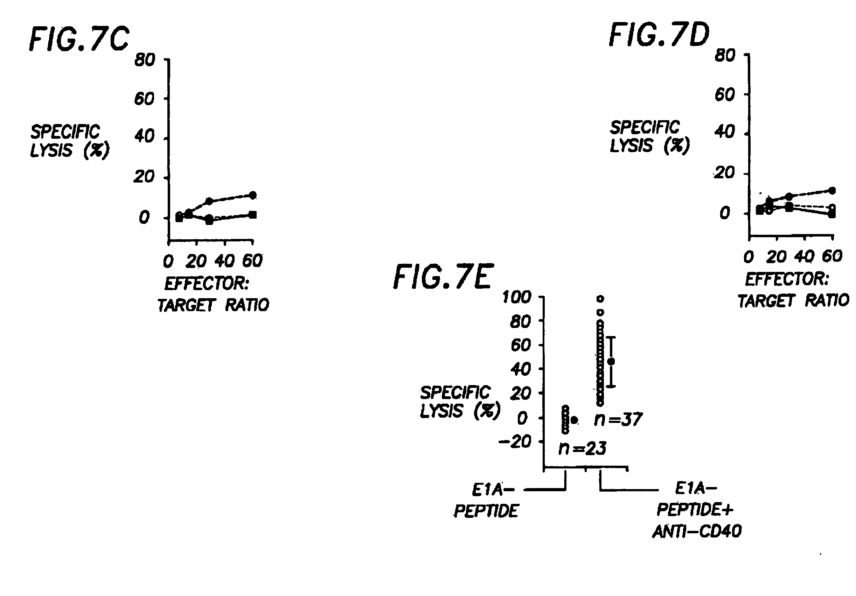

### Figure 7: CTL-tolerance induction is reverted into CTL-priming after CD40-triggering in vivo

Wild type C57BL/6 mice (**a, b**) and H-2^{b} CD40 ^{-/-} mice (**c, d**) were injected s.c. with 20 µg E1A-peptide in IFA alone (c), in combination with a rat IgG2a control antibody (**a**), or in combination with the anti-CD40 mAb FGK-45 (**b, d**). Bulk cultures from these mice were tested for E1A-specific cytotoxicity on target cells pulsed with the E1A-peptide (-■-), the HPV16 E7-peptide (-O-) or Ad5E1 transformed tumor cells (-◆-). Specific lysis of representative bulk cultures at different E/T ratios is shown. This experiment has been repeated 18 (B6 mice) and 2 (CD40-/- mice) times, respectively, with comparable results. In (**e**) the % specific lysis of 23 respectively 37 bulk CTL cultures derived from B6 mice injected with E1A-peptide in IFA alone (left) or in combination with the anti-CD40 mAb (right) at an E/T of 60 is shown. Mean plus standard deviation of each group are shown (E1A versus E1A+anti-CD40: p<0.01, student t-test).

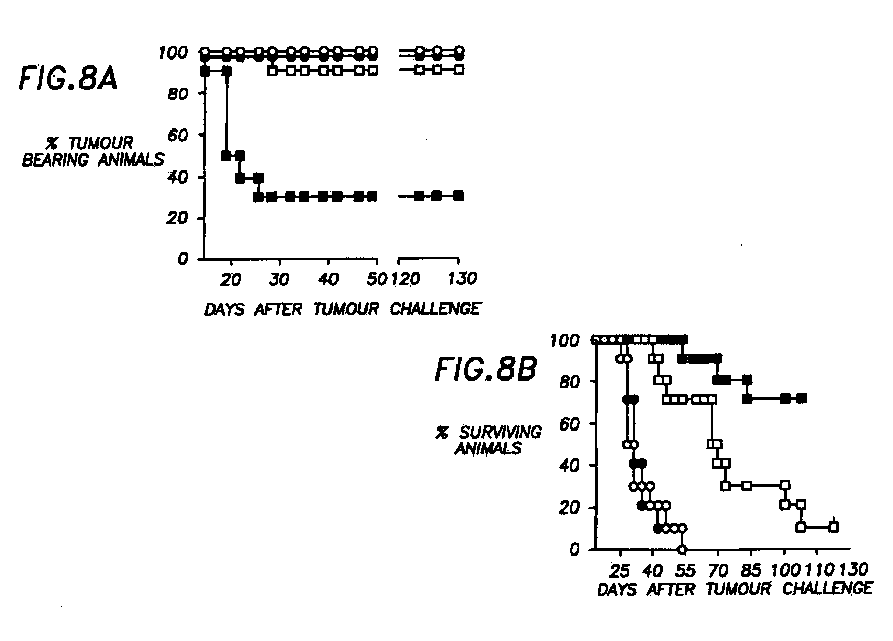

### Figure 8: Therapy of HIPV16 E6 and E7 transformed cells by combination treatment of peptide together with in vivo CD40 triggering

Mice were injected s.c with 25.000 HPV16 transformed syngeneic cells (TC-1). C57BL/6 mice were left untreated (-O-) or after 6 days received 100 µg HPV16 E7-peptide i.p. in IFA (-□-), 100 µg HPV16 E7-peptide i.p. in IFA in combination with the anti-CD40 mAb FGK-45 (-■-) or a control peptide i.p. in IFA in combination with the anti-CD40 mAb FGK-45 (-•-). The percentage of tumor bearing mice is depicted for different treatment groups (n=10) in (**a**). The differences between the group treated with the HPV16-peptide plus the anti-CD40 mAb and the other three groups were statistically significant (p<0.01) (Log-Rank test). In (**b**) the percentage of surviving animals is shown (E7-peptide-treated group vs E7-peptide plus anti-CD40-treated group: p = 0.002, Log-Rank test).

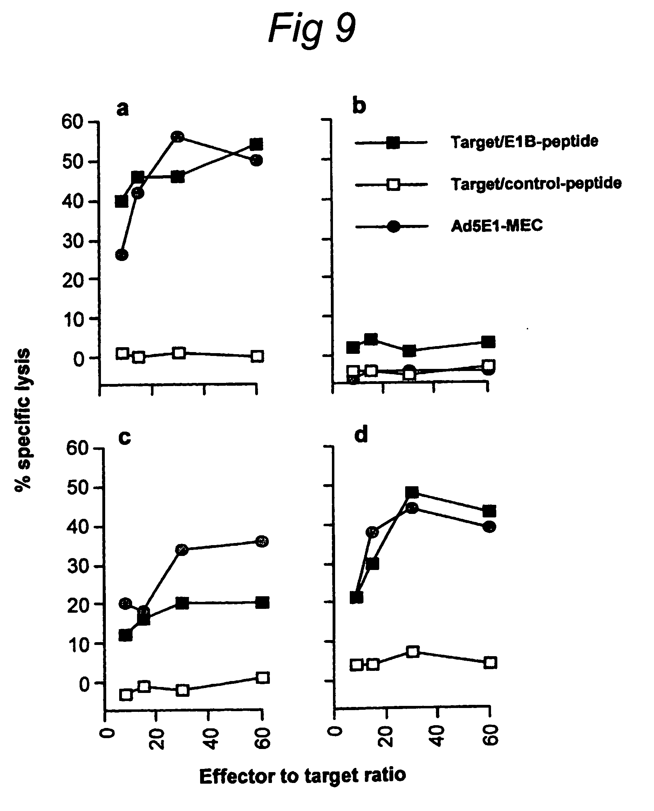

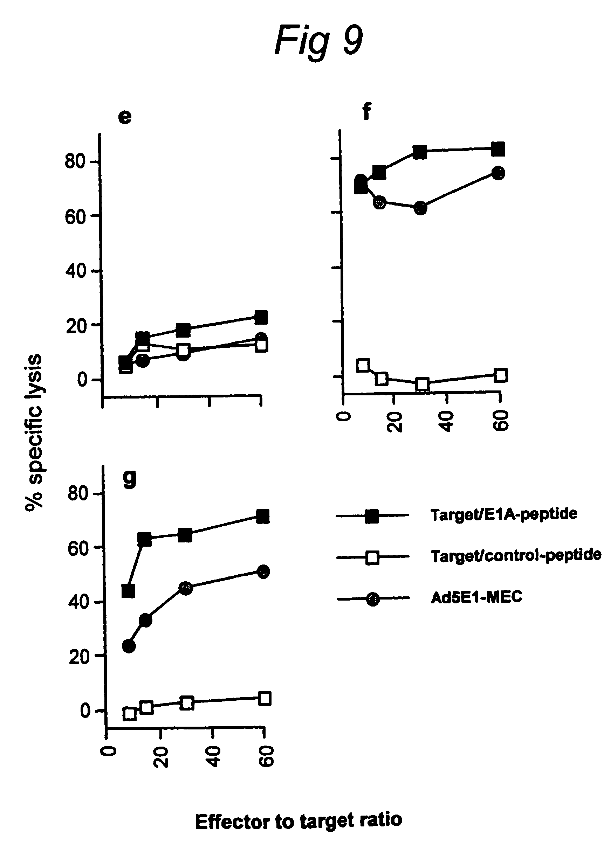

### Figure 9: In vivo triggering though 4-1BB restores priming of Ad5-specific CTL in the absence of T cell help

B6 mice, either non-depleted (a) or depleted for CD4⁺ T cells (b, c, d) were vaccinated with 10⁷ TAP k.o. Ad5E1MEC s.c. in combination with systemic doses of control Ab (b), anti-CD40 Ab (c) or anti-4-1BB Ab (d). In a separate experiment, normal B6 mice received a dose of 20 µg E1A peptide in IFA either in combination with systemic doses of control Ab (e), anti-CD40 (f) or anti-4-1BB Ab (g). Splenocytes for immunised mice were restimulated in vitro for 6 days and subsequently tested for antigen-specific cytotoxicity on target cells loaded with the E1B-peptide (panels a-d) or the E1A-peptide (panels e-g)(■), the HPV16 E7-peptide (□) or Ad5E1MEC (grey O). The stimulatory effects of 4-1BB Ab on priming of Ad5-specific CTL by tumor cells and the peptide vaccine have each been reproducibly observed in 3 separate experiments.

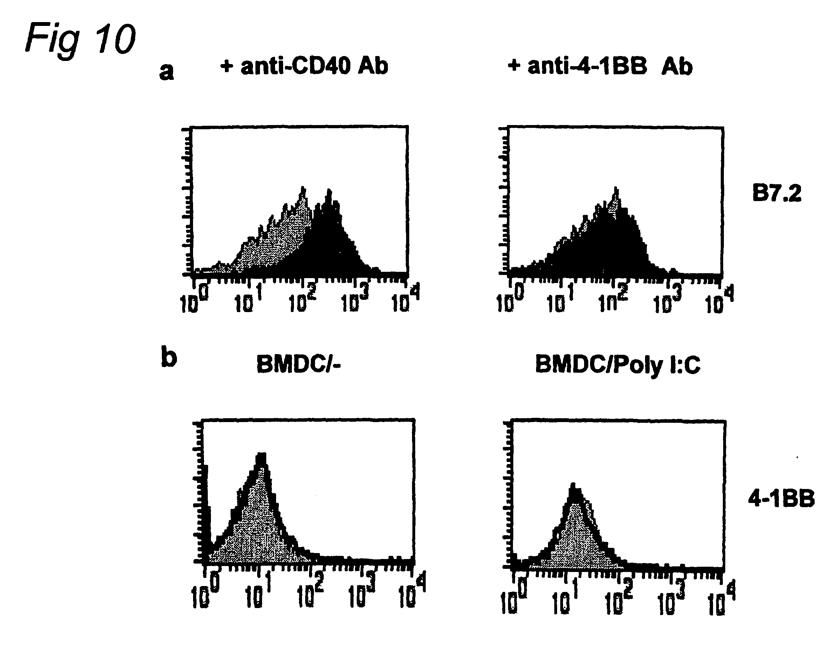

### Figure 10: Systemic administration of anti-4-1BB Ab does not induce DC activation in vivo

a: B6 mice were injected twice with 100 µg of control Ab (light grey histograms), anti-CD40 Ab (dark grey, left panel) or the anti-4-1BB Ab (dark grey, right panel). Three days later spleen cells were isolated and stained with anti-CD 11c and anti-CD86. Depicted are the CD86 expression levels on CD11c⁺ cells in the spleen. b: B6 bone marrow was isolated and cultured in vitro for 7 days in GM-CSF-containing medium. On day 7, cells were activated by addition of 10 µg/ml Poly I:C or left untreated. 48h later cells were analysed for 4-1BB expression (black line) or stained with a control Ab (filled light grey). Activation of Poly I:C-treated BMDC was confirmed by measuring CD86 expression (not shown).

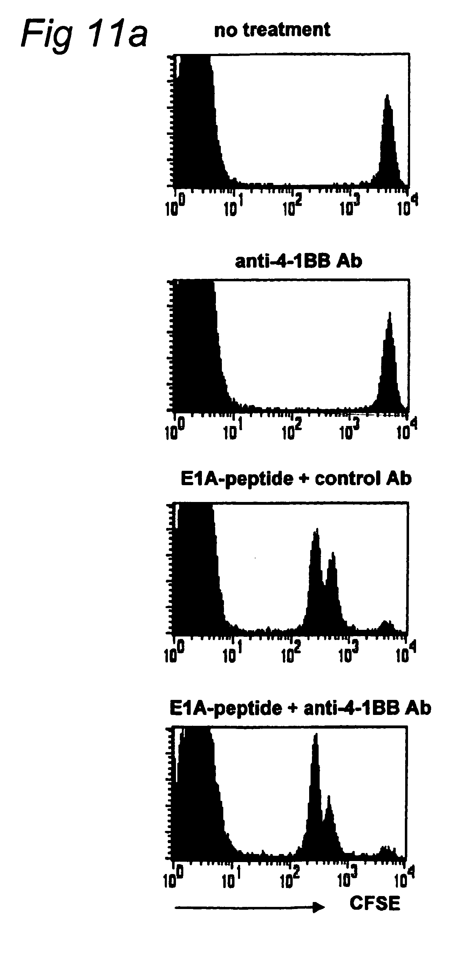

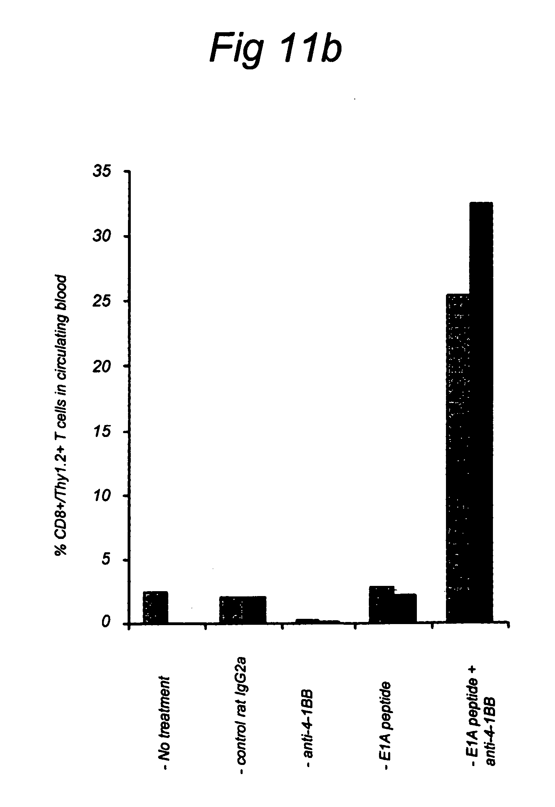

### Figure 11: Costimulation through 4-1BB is antigen-dependent and results in increased survival of antigen-specific T cells

a: B6 mice were injected with 5 x 10⁶ Ad5E1A-specific TCR transgenic CD8⁺ T cells that were labelled with CFSE in vitro. Three days later these mice received a combination of E1A peptide vaccine and Ab as indicated in the figure. Peptide vaccination involved a single s.c. 20 µg dose in IFA, whereas 100ug doses of Ab were administered i.p. on three consecutive days following peptide vaccination. At day 3 after injection of the peptide, spleen cells were isolated and analysed by FACS for the presence of CD8⁺/CFSE⁺ T cells. The histogram shows the CFSE intensity of CD8⁺ T cells. b: B6 Thy1.1 mice were injected with 5 x 10⁶ E1A-peptide specific TCR transgenic CD8⁺ T cells of B6 Thy1.2 origin. Three days later these mice received a combination of E1A peptide vaccine and Ab as indicated in the figure (doses and timing as above). 11 days after injection of the peptide vaccine, blood samples were taken and analysed by FACS for the presence of CD8⁺/Thy1.2⁺ T cells (of E1A-peptide specific TCR transgenic origin).

### Figure 12: 4-1BBL is upregulated on activated DC. B6 bone marrow was isolated and cultured in vitro for 7 days in GM-CSF-containing medium.

On day 7, cells were left untreated (a) or activated with 10 µg/ml Poly I:C (b) or 2 µg/ml murine CD40L-trimer (c). After 48h, cells were stained for CD11c in combination with either control Ab (black lines) or anti-4-1BBL Ab (grey filled lines). The histograms show the CD11c⁺ cell population.

### Figure 13: Both CD28 and 4-1BB contribute to cross priming of tumor-specific CTL

B6 mice were left untreated or were immunised s.c. with 10⁷ Ad5E1-transformed tumor cells in combination with blocking anti-4-1BBL Ab, isotype control Ab and/or CTLA4-Ig as indicated in the figure. Spleen cells were isolated 10 days after immunisation and restimulated in vitro for 6 days. Bulk cultures were analysed for the presence of antigen-specific CTL by double staining with anti-CD8 antibody and an anti-INFy Ab after overnight stimulation with 1 µg/ml of the E1B-encoded CTL epitope. The data shown are an average of three independent experiments. Error bars show S.E.M. values.

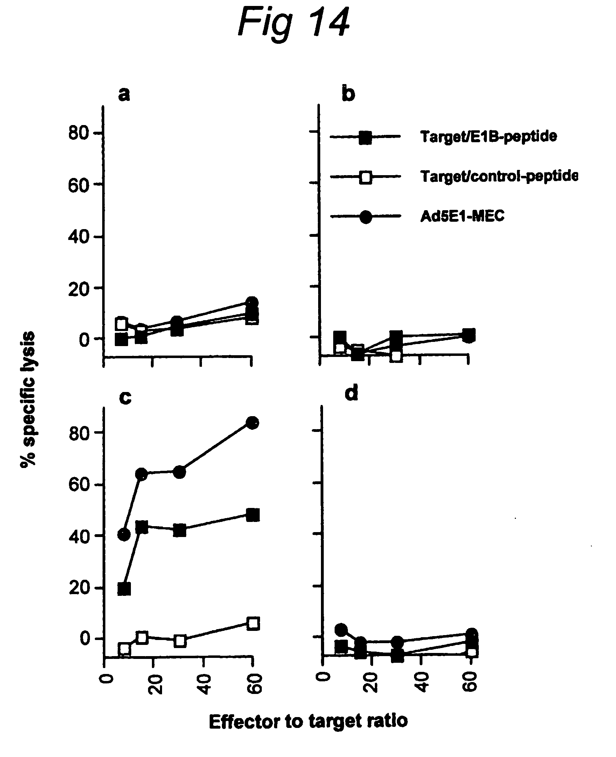

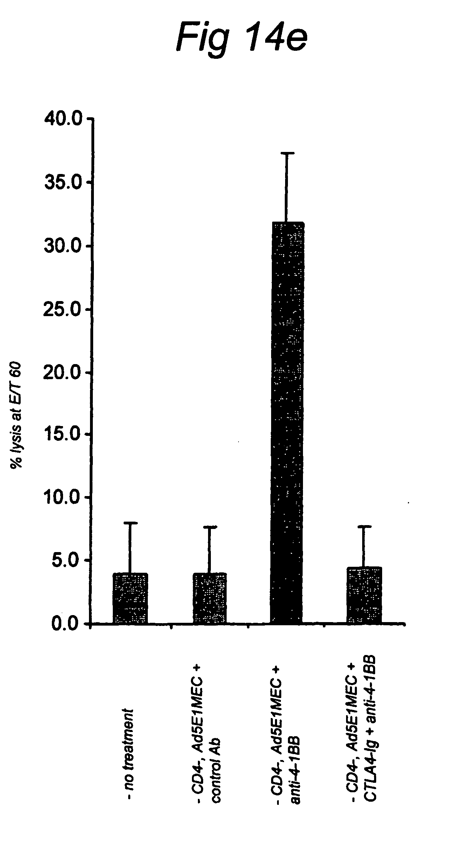

### Figure 14: Costimulation through CD28 signalling is prerequisite for efficacy of anti-4-1BB Ab treatment

B6 mice, either non-depleted (a) or depleted for CD4⁺ cells (b-d), were vaccinated with 10⁷ Ad5E1MEC s.c. in combination with control Ab (b), anti-4-1BB Ab (c), or anti-4-1 BB Ab in combination with CTLA4-Ig (d). Splenocytes were harvested 10 days after vaccination and restimulated in vitro for 7 days. Bulk cultures were tested for E1B-specific cytotoxicity on target cells loaded with the E1B-peptide (■), the HPV16 E7-peptide (□) or Ad5E1-transformed tumor cells (grey O). (e) The average lysis of these bulk cultures on E1B-peptide loaded target cells at an effector to target ratio of 60:1 is depicted. Error bars represent S.E.M. values from 6 mice over two independent experiments.

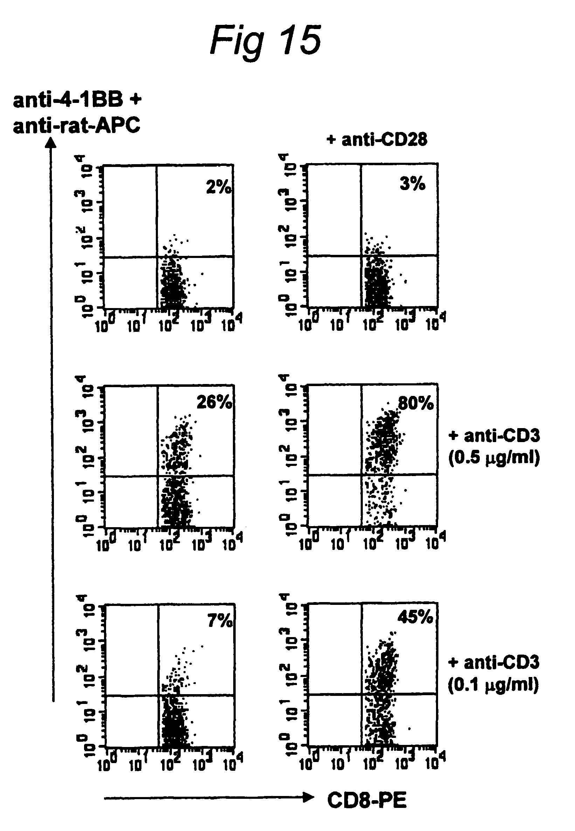

### Figure 15: CD28 costimulation facilitates induction of 4-1BB expression on TCR-triggered T cells

B6 spleen cells were cultured in the presence of the indicated amounts of plate-bound anti-CD3 Ab and/or plate-bound anti-CD28 Ab (5 µg/ml). After 24h CD8⁺ T cells were analysed for the expression of 4-1BB.

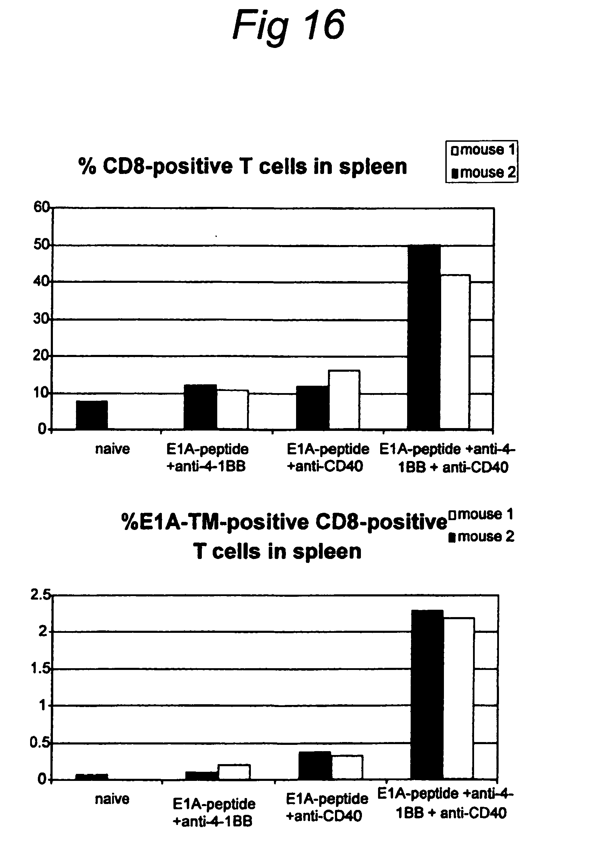

### Figure 16: 4-1BB signalling synergises with CD40 signalling in inducing E1A-specific CTL in vivo

C57BL/6 mice were vaccinated with E1A-peptide s.c. with 100 micrograms of anti-CD40 Ab FGK-45, anti-4-1BB Ab 3H3, or a combination of both i.p in PBS. Ten days later spleen cells were isolated and analysed fro the presence of E1A-specific CTL by staining with CD8-APC and D^{b}-E1A tetramers. Depicted are the absolute numbers of CD8⁺ T cells in the spleen (upper panel) and the percentage of E1A-specific CTL within this CD8⁺ population (lower panel).

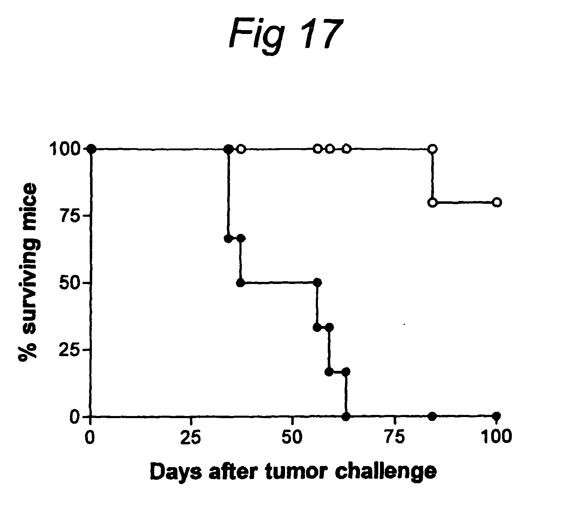

### Figure 17: CD40-ligation in vivo leads to the elimination of established tumors

Mice were injected with Ad5E1A-expressing tumor cells. At the time that mice had developed palpable tumors, mice were left untreated (closed circles) or were injected i.v. with a CD40-activating antibody (open circles). Mice were sacrificed when the tumor size exceeded 1 cm³ to avoid unnecessary suffering.

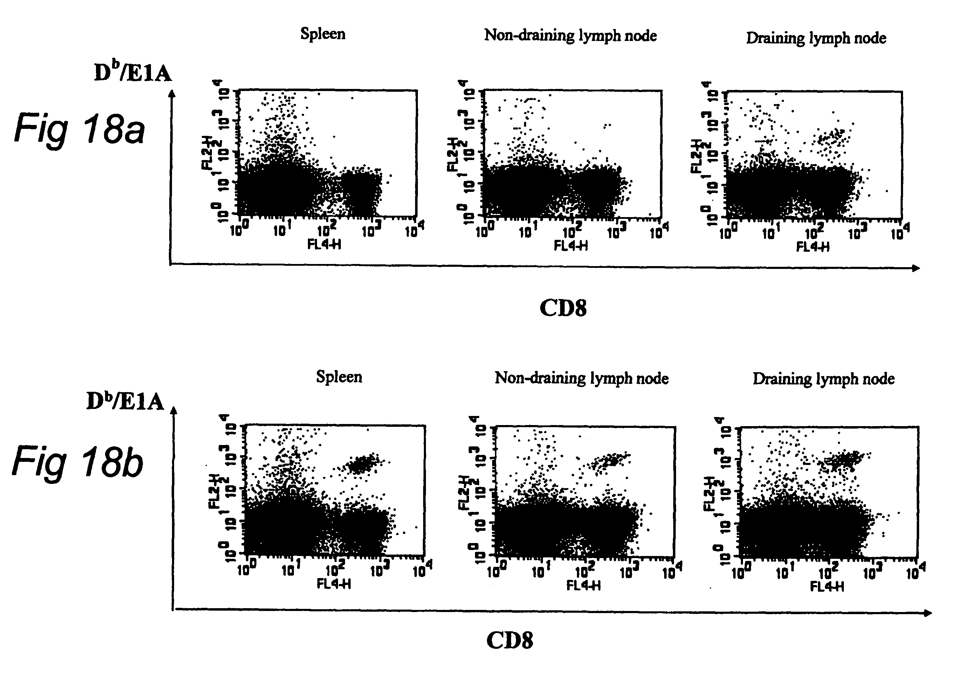

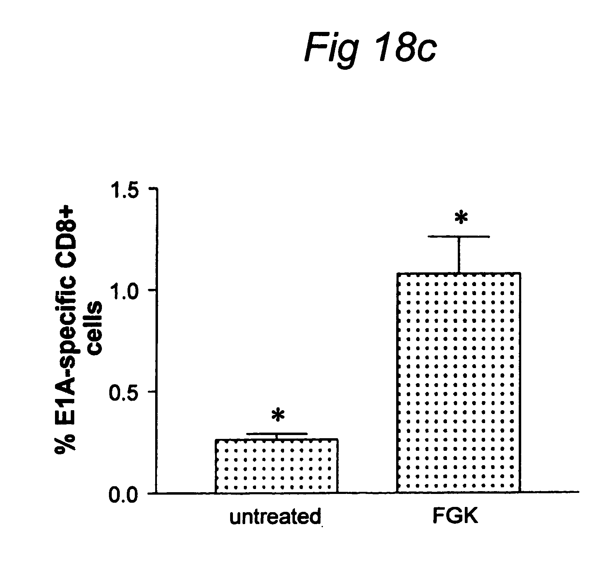

### Figure 18: In vivo CD40-activation leads to systemic spread of tumor-specific CTL

Mice were injected with Ad5E1A-expressing tumor cells. At the time that mice had developed palpable tumors, they were either left untreated or were treated with anti-CD40 mAb. Ten days after anti-CD40 injection, mice were sacrificed and FACS-analysis was performed on blood, spleen, and lymph nodes. In over 30% of untreated mice (n=23), E1A-specific CTL were detected in tumor draining lymph nodes only (A), whereas no E1A-specific CTL were observed in the other animals of this group (not shown). In contrast, E1A-specific CTL were readily detectable in tumor-bearing mice treated with anti-CD40 mAb (n=29; B). C: The number of E1A-specific CD8+ T-cells as a percentage of the total CD8+ T cell pool in blood is depicted (mean + SEM). One representative experiment out of 4 is shown. *Student's T-test: p < 0.01

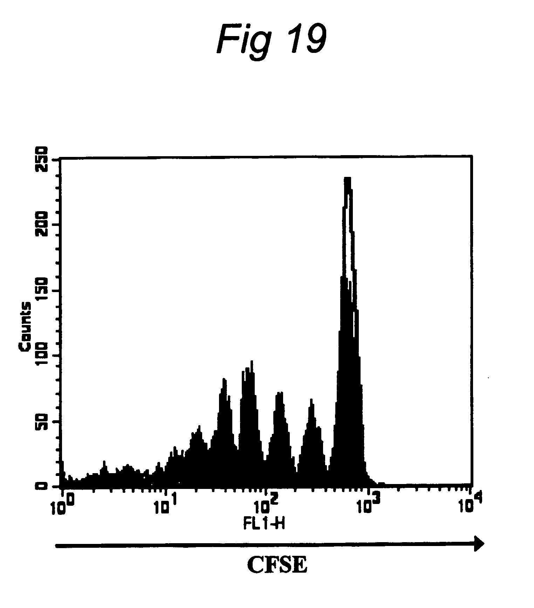

### Figure 19: The presence of the E1A-derived CTL-epitope is detected in the tumor-draining lymph node only

4*10⁶ CFSE-labeled E1A-specific transgenic T-cells were injected i.v. into mice bearing an Ad5E1A-expressing tumor. Seven days later the spleen, tumor-draining lymph node and a non-draining lymph node were taken and analysed for division of E1A-specific CTL by FACS. The results obtained from the tumor-draining lymph node (black area) and the spleen (gray line) are shown and is representative of 12 mice. Results were comparable for anti-CD40-treated mice and untreated mice.

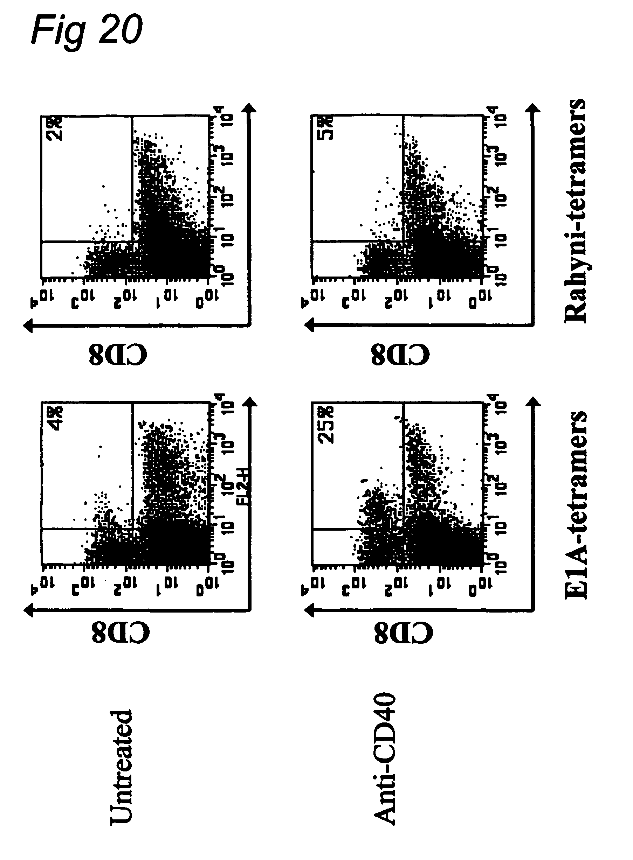

### Figure 20: In vivo CD40-triggering leads to tumor infiltration of Ad5E1A-specific CTL

Mice were injected with Ad5E1A-expressing tumor cells. At the time when palpable tumors had developed, mice were left untreated or treated i.v. with the CD40-activating antibody. Five days thereafter, mice were sacrificed and tumorinfiltration of E1A-specific CTL was determined by FACS-analysis. Numbers in the upper right corner represent percentage of TM-positive cells of the total pool of CD8⁺ cells.

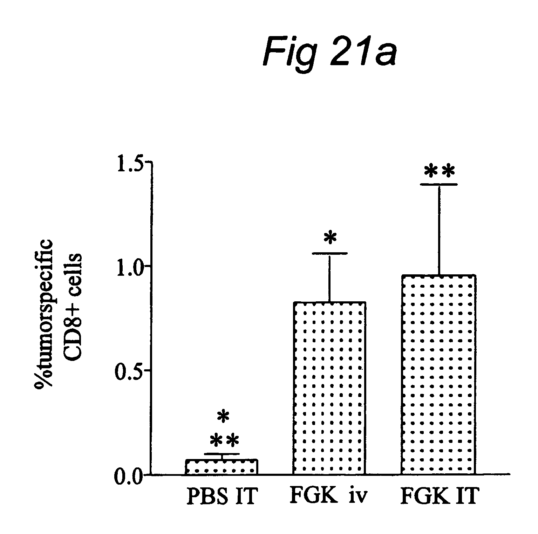

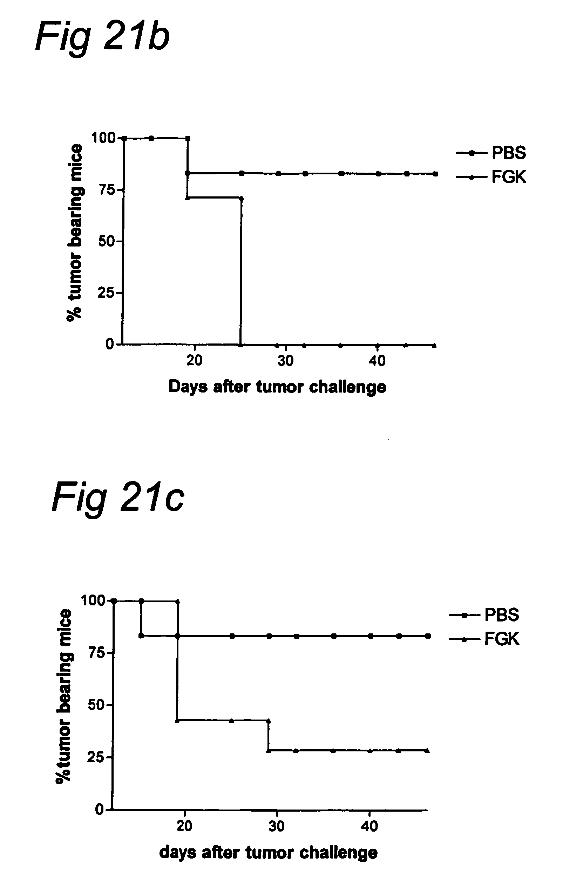

### Figure 21: Local CD40-activation causes systemic anti-tumor immunity

Mice were injected with Ad5E1A-expressing tumor cells in both flanks. At the time when palpable tumors had developed on both flanks, anti-CD40, or PBS as control was given intratumoral in one of the tumors. A) Ten days after treatment blood samples were taken and analysed by FACS to determine the presence of Ad5E1A-specific CTL. Results represent the number of E1A-specific CD8⁺ T-cells as a percentage of the total CD8-pool. (mean + SEM, n=7). *student's T-test: p<0,05; **student's T-test: p>0,01; no significant difference between intratumoral and i.v. anti-CD40 treatment. B) Percentage of mice bearing a tumor in the flank treated. p<0.01C) Percentage of mice bearing a tumor in the not treated flank. p=0.05

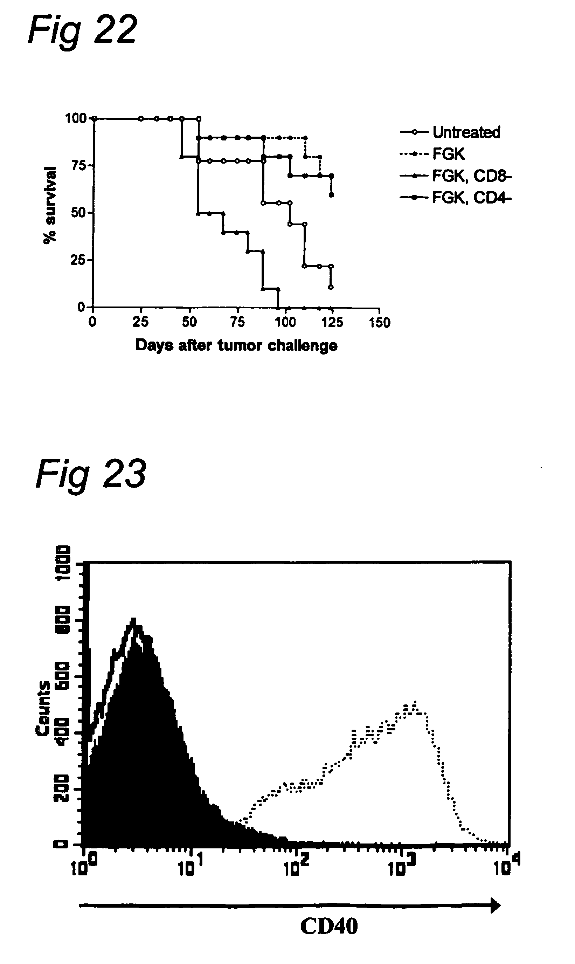

### Figure 22: CD8⁺ T-cells but not CD4⁺ T-cells are crucial for tumor eradication after in vivo CD40-ligation

Mice were injected with AdSE1A-expressing tumor cells. At the time that mice had developed palpable tumors, treatment was started. Mice received no treatment, anti-CD40 mAb-treatment or a combination of anti-CD40 mAb with a CD8- or a CD4-depleting Ab. Mice were sacrificed when the tumor size exceeded 1 cm³ to avoid unnecessary suffering.

### Figure 23: Ad5E1A-expressing tumors do not express CD40

FACS-analysis was performed with cells stained with PE-labeled anti-CD40-antibody (black line) or isotype control antibody (gray area). LPS-activated dendritic cells were used as a positive control (dotted line).

### Detailed Description

The CD40 binding molecules of the invention can be made by conventional production and screening techniques. A rat and a hamster anti-mouse CD40 monoclonal antibody ("Mabs") are each described in *Nature* 393: 474-77 (1998) and are available commercially (Pharmingen, Inc., CA). The anti-mouse CD40 MAb, designated FGK45, which is used in the experiments described below, is described by Rolink. A. *et al., Immunity* 5, 319-330 (1996). Agonistic anti-4-1 BB antibodies are described in Hurtado et al. (1997) and Shuford et al. (1997). Anti-human CD40 MAbs, can be made following techniques well-known in the art, and described by G. Köhler and C. Milstein (*Nature,* 1975: 256: 495-497). MAbs can be raised by immunizing rodents (*e.g.* mice, rats, hamsters and guinea pigs) with either native CD40 as expressed on cells or purified from human plasma or urine, or recombinant CD40 or its fragments, expressed in a eukaryotic or prokaryotic system. Other animals can be used for immunization, *e.g.* non-human primates, transgenic mice expressing human immunoglobulins and severe combined immunodeficient (SCID) mice transplanted with human B lymphocytes. Hybridomas can be generated by conventional procedures by fusing B lymphocytes from the immunized animals with myeloma cells (e.g. Sp2/0 and NS0), as described by G. Köhler and C. Milstein *Id.* In addition, anti-CD40 MAbs can be generated by screening of recombinant single-chain Fv or Fab libraries from human B lymphocytes in phage-display systems. The specificity of the MAbs to CD40 can be tested by enzyme linked immunosorbent assay (ELISA), Western immunoblotting, or other immunochemical techniques. The activating activity of the antibodies on CTLs, in combination with a CTL-activating peptide, can be assessed using the assays described in the Examples below.

For treating humans, the anti-CD40 MAbs would preferably be used as chimeric, Deimmunised, humanized or human antibodies. Such antibodies can reduce immunogenicity and thus avoid human anti-mouse antibody (HAMA) response. It is preferable that the antibody be IgG4, IgG2, or other genetically mutated IgG or IgM which does not augment antibody-dependent cellular cytotoxicity (S.M. Canfield and S.L. Morrison, *J. Exp. Med.,* 1991: 173: 1483-1491) and complement mediated cytolysis (Y.Xu et al., *J. Biol. Chem.,* 1994: 269: 3468-3474; V.L. Pulito et al., *J. Immunol.,* 1996; 156: 2840-2850).

Chimeric antibodies are produced by recombinant processes well known in the art, and have an animal variable region and a human constant region. Humanized antibodies have a greater degree of human peptide sequences than do chimeric antibodies. In a humanized antibody, only the complementarity determining regions (CDRs) which are responsible for antigen binding and specificity are animal derived and have an amino acid sequence corresponding to the animal antibody, and substantially all of the remaining portions of the molecule (except, in some cases, small portions of the framework regions within the variable region) are human derived and correspond in amino acid sequence to a human antibody. See L. Riechmann et al., *Nature,* 1988; 332: 323-327; G. Winter, *United States Patent* No. 5,225,539; C.Queen et al., U.S. patent number 5,530,101.

Deimmunised antibodies are antibodies in which the T and B cell epitopes have been eliminated, as described in in International Patent Application PCT/GB98/01473. They have reduced immunogenicity when applied *in vivo.*

Human antibodies can be made by several different ways, including by use of human immunoglobulin expression libraries (Stratagene Corp., La Jolla, California) to produce fragments of human antibodies (VH, VL, Fv, Fd, Fab, or (Fab')₂, and using these fragments to construct whole human antibodies using techniques similar to those for producing chimeric antibodies. Human antibodies can also be produced in transgenic mice with a human immunoglobulin genome. Such mice are available from Abgenix, Inc., Fremont, California, and Medarex, Inc., Annandale, New Jersey.

One can also create single peptide chain binding molecules in which the heavy and light chain Fv regions are connected. Single chain antibodies ("ScFv") and the method of their construction are described in U.S. Patent No. 4,946,778. Alternatively, Fab can be constructed and expressed by similar means (M.J. Evans et al., *J. Immunol. Meth.,* 1995; 184: 123-138). All of the wholly and partially human antibodies are less immunogenic than wholly murine MAbs, and the fragments and single chain antibodies are also less immunogenic. All these types of antibodies are therefore less likely to evoke an immune or allergic response. Consequently, they are better suited for *in vivo* adminstration in humans than wholly animal antibodies, especially when repeated or long-term adminstration is necessary. In addition, the smaller size of the antibody fragment may help improve tissue bioavailability, which may be critical for better dose accumulation in acute disease indications, such as tumor treatment.

Based on the molecular structures of the variable regions of the anti-CD40 mAbs or the known CTL-activating peptides, one could use molecular modeling and rational molecular design to generate and screen molecules which mimic the molecular structures of the binding region of the antibodies or the peptides, respectively, and activate CTLs. These small molecules can be peptides, peptidomimetics, oligonucleotides, or other organic compounds. The mimicking molecules can be used for treatment of cancers and infections. Alternatively, one could use large-scale screening procedures commonly used in the field to isolate suitable molecules from libraries of compounds.

In one aspect the disclosure thus relates to a composition comprising an agonistic anti-4-1BB antibody, or fragment thereof which stimulates the 4-1BB receptor. Preferably the composition further comprises an agonistic anti-CD40 antibody, or fragment thereof which stimulates the CD40 receptor.

In a further aspect the disclosure relates to a composition comprising either (a) an agonistic anti-CD40 antibody, or fragment thereof which stimulates the CD40 receptor, and which composition further comprises one or more molecules selected from proteins, polypeptides, peptides, viruses, or nucleic acid sequences having or encoding a class I MHC or class II MHC-restricted T cell epitope.

Methods for selecting and producing suitable class I MHC and/or class II MHC-restricted T cell epitope are e.g. described in WO07/41440 and in WO02/070006. Preferably the suitable class I MHC and/or class II MHC-restricted T cell epitope are peptides. Preferably the peptide have a length of at least 8 amino acids, more preferably at least 22 amino acids. Preferred peptides have a length 8-10, more preferably 22 - 45 amino acids.

In a further aspect the disclosure relates to the use of (a)_an agonistic anti-4-1BB antibody, or fragment thereof which stimulates the 4-1BB receptor, (b) an agonistic anti-CD40 antibody, or fragment thereof which stimulates the CD40 receptor, (c) or both in the manufacture of a medicament for the induction of systemic T cell immunity against an antigen. The medicament may further comprise one or more molecules selected from proteins, polypeptides, peptides, viruses, or nucleic acid sequences having or encoding a class I MHC or class II MHC-restricted T cell epitope in the manufacture of a medicament for the induction of systemic T cell immunity against an antigen. The antigen preferably is a class I MHC or class II MHC-restricted T cell epitope. The antigen preferably is an antigen of a tumor or tumor cells, more preferably the antigen is a tumor-specific antigen. Alternatively, the antigen may be an antigen of an infectious agent. A preferred antigen is an HPV antigen, more preferably the HPV antigen is a peptide having the sequence RAHYNIVTF (SEQ ID NO:3). In another aspect the invention relates to the use of an agonistic anti-CD40 antibody, or fragment thereof which stimulates the CD40 receptor in the manufacture of a medicament for the treatment of a tumor or an infectious agent by induction of systemic T cell immunity, whereby the treatment does not comprise immunisation (e.g. vaccination) with an antigen of the tumor or infectious agent.

The dosage for the molecules of the invention can be readily determined by extrapolation from the *in vitro* tests and assays described below, or from animal experiments or from human clinical trials. The molecules of the invention would be preferentially administered by injection, in the case of antibodies or proteins, although certain small molecules may be suited for oral administration. The assays and tests demonstrating the efficacy of the invention are described below.

### Examples

### Example 1: Signaling through CD40 can replace CD4⁺ helper T cells in CTL priming

A well characterized model system to probe the mechanism of T-cell help for the primary activation of CD8+ CTL responses *in vivo* was used. C57BL/6 (with the major histocompatibility complex (MHC) H-2^{b}) mice immunized with allogenic BALB/c (H-2^{d}) mouse embryo cells (MECs) expressing the human adenovirus type 5 early region 1 (Ad5EI-BALB/c MECs) generated strong CTL responses against an H-2D^{b}-restricted epitope of the adenovirus EIB protein (EIB₁₉₂₋₂₀₀) (Figure 1a). As the allogeneic H-2^{d} MHC molecules expressed by the Ad5EI-BALC/c MECs cannot prime H-2^{b}-restricted host CTLs, generation of E1B-specific CTLs must require cross-priming, that is, the uptake and H-2^{b}-restricted re-presentation of antigen by host APCs. Cross-priming of EIB-specific CTLs is strictly helper-dependent (Figure 1b), as mice depleted of CD4+ T-helper (Tₕ) cells before immunization no longer mounted an E1B-specific CTL response.

To investigate whether signalling through CD40 can replace CD4⁺ helper T cells in CTL priming, mice were depleted of CD4⁺ T cells *in vivo* before immunization with Ad5E1BALB/c MECs. One day after immunization, the mice received a single injection of the activating antibody anti-mouse CD40 mAb FGK45, or of an isotype-matched control antibody. Administration of FGK45 to CD4-depleted, immunized mice resulted in the efficient restoration of E1B-specific CTL responses (Figure 2a) whereas treatment with the control antibody did not (Figure 2b). Priming of E1B-specific CTLs was not detected in naïve mice treated with FGK45 alone (not shown). To address the possibility that the effect of FGK45 was mediated through remaining D4⁺ cells that were not depleted by treatment with the anti-CD4 antibody, B6 I-A^{b} knockout mice, which lack mature functional CD4⁺ peripheral T cells, were immunized with the Ad5EI-BALB/c MECs. The response to immunization in these mice mirrors that seen in the CD4-depleted mice, in that E1B-specific CTLs were detectable only in mice receiving the CD40-activating antibody (Figure 2c), and not in those receiving the control antibody (Figure 2d).

It was also studied whether the requirement for anti-CD40 antibodies in priming of CTLs in CD4-depleted mice could be replaced by bacterial lipopolysaccharide (LPS) (50 µg intravenous), a potent inducer of proinflammatory cytokines, or by administration of IL-2 (1 x 10⁵ units in incomplete Freund adjuvant, subcutaneous) following immunization with Ad5EI-BALB/c MECs. Whereas CD4-depleted mice treated with FGK45 exhibited strong E1B-specific CTL activity, neither LPS or IL-2 treatment resulted in detectable CTL priming (not shown).

Ligation of CD40 on B cells upregulates their costimulatory activity, suggesting a role for these cells in the restoration of CTL priming by treatment with CD40 activating antibodies. To address this question, B6 □MT mice, which lack mature B cells, were immunized with the allogeneic Ad5EI-BALB/c MECs. Cross-priming of E1B-specific CTLs did not require mature B cells (Figure 3a). However, when depleted of CD4⁺ cells, the B-cell deficient mice did not generate an E1B-specific CTL response (Figure 3b). Activation through CD40 with the FGK45 monoclonal antibody completely restored the capacity of CD4-depleted □MT mice to prime E1B-specific CTLs (Figure 3c). Thus B cells are not required as APCs or accessory cells for cross-priming in this model system, nor are they required for CD40-mediated restoration of cross priming of CTLs in the absence of CD4⁺ helper T cells. These results demonstrate that activation of bone marrow derived APC through CD40 can bypass the requirement for CD4⁺ T-helper cells in the cross-priming of E1B-specific CTLs.

### Example 2: Blocking the ability of CD4⁺ helper T cells to interact with APC through the CD40L-CD40 pathway prevents antigen-specific CTL responses in normal mice

If the CD40L-CD40 interaction represents the physiological pathway used by CD4⁺ helper T cells to help CTLs, blocking the ability of the CD4⁺ T cells to interact with APC through CD40L-CD40 interaction would be expected to diminish priming of E1B-specific CTL responses in normal mice. B6 mice were immunized with Ad5E1-BALB/c MECs and then treated with either the CD40L-blocking antibody MR1, or control antibody. Blockade of CD40L results in drastically reduced E1B-specific CTL responses (Figure 4a) compared to the efficient CTL priming seen in mice receiving the control antibodies (Figure 4b). The priming defect induced by CD40L blockade was fully restored following CD40 signalling by FGK45 (Figure 4c). Thus the defect in CTL-priming induced by CD40L blockade lies in the failure of T_{H} cells to transmit, rather than to receive, CD40L-mediated signals.

### Example 3: E1A-specific CTL unresponsiveness after peptide administration

A previously described model system has been used (Toes et al., *J. Immunol.* 156:3911 (1996)). It has been shown that s.c. vaccination with the Ad5E1A-derived CTL epitope SGPSNTPPEI (SEQ ID NO: 2) in IFA prevents mice from controlling the outgrowth of Ad5E 1 A-expressing tumors. This indicates that the E1A/IFA vaccine induced suppression rather than induction of E1A-specific CTL immunity. Moreover, administration of the E1A/IFA vaccine to T cell receptor (TCR)-transgenic mice, which express the TCR α and β chains of an E1A-specific CTL clone, strongly suppressed tumor-specific CTL-mediated immunity. These experiments examined the effects of peptide administration on a monoclonal CTL population. To establish whether s.c. E1A-peptide vaccination also induces E1A-specific CTL tolerance at the polyclonal CTL level, wild type (wt) C57BL/6 mice were injected with either E1A-peptide (Figure 5a and 5b) or a control peptide Figure 5c and 5d). One day later the mice were boosted with a syngeneic cell line expressing high levels of the E1A-peptide at its surface (Figure 5b and 5d). Injection of this cell line into mice primed with the control peptide readily induces E1A-specific immunity (Figure 5d). However, the ability of mice to mount E1A-specific CTL responses was abrogated after injection of the E1A/IFA vaccine (Figure 5b). These data indicate that injection of the E1A-peptide not only leads to E1A-specific tolerance in TCR-transgenic mice but also in mice expressing a polyclonal E1A-specific T cell repertoire.

Since s.c. injection of the E1A/IFA vaccine leads to systemic CTL tolerance, it was investigated whether the E1A-peptide is dispersed systemically and presented to precursor CTL in the periphery. Therefore, mice were injected s.c. with the E1A-peptide or Human Papilloma Virus (HPV) 16 E7-derived control peptide emulsified in IFA. Spleen cells from these mice were isolated 16h later and used as stimulator cells for an E1A-specific CTL clone *in vitro.* Splenocytes from mice injected with the E1A-peptide s.c. induced specific proliferation, whereas splenocytes from mice injected with the E7-peptide s.c. failed to do so (Figure 6). Moreover, a control CTL clone did not proliferate on spleen cells derived from E1A-injected mice (data not shown). Thus, these data indicate that the E1A-peptide injected s.c. in IFA is systemically presented in the periphery by, amongst others, splenocytes.

In view of the tolerizing effects described above of the E1A-peptide vaccine, there was a question whether CD40-triggering *in vivo* is sufficient to prevent peripheral tolerization of CTL and to restore CTL priming. Therefore, it was investigated whether injection of tolerizing peptides combined with *in vivo* CD40 triggering could prevent the induction of peripheral CTL tolerance leading to tumor-specific CTL immunity.

In Examples 1 and 2, it has been shown that CD40-triggering *in vivo* can replace the requirement for CD4⁺ T helper cells in priming of helper-dependent CTL responses. Since CD4⁺ T cell-mediated helper activity has been implicated in the prevention of peripheral CTL tolerance induction, the inventors addressed the question whether CD40-triggering *in vivo* is sufficient to prevent peripheral E1A-specific CTL tolerization. To this end, mice were injected with the E1A/IFA vaccine in combination with the activating anti-CD40 mAb FGK-45. Mice that received this combination mounted strong E1A-specific CTL responses (Figure 7b and 7e), whereas mice that received the E1A/IFA vaccine (Figure 7e) or mAb alone did not (not shown). The combination of E1A/IFA vaccine and anti-CD40 mAb failed to elicit CTL in CD40-deficient mice (Figure 7c and 7d). Furthermore, co-injection of the E1A/IFA vaccine with an isotype-matched control mAb (Figure 6a) or IL-2 failed to convert CTL tolerance induced by the E1A/IFA vaccine into CTL priming (not shown). The range and variation of responses to the E1A-epitope in E1A-peptide only, or E1A-peptide plus anti-CD40-vaccinated animals, is shown in Figure 7e. Thus, systemic CD40 activation can reverse peptide-induced peripheral CTL tolerance into peptide and tumor-specific CTL mediated immunity.

The induction of E1A-specific immunity strongly correlated with the presence of CD8⁺ T cells in the spleen of vaccinated mice that stained with PE-conjugated H-2-D^{b}-tetramers containing the E1A-peptide (D^{b}/E1A). Within 10 days after vaccination, CD8⁺ T cells staining with D^{b}/E1A tetramers could be detected by flow cytometry in mice injected with E1A-peptide and the anti-CD40 mAb, but not in mice injected with E1A-peptide alone (not shown). In the mice injected with E1A-peptide, the percentage of CD8⁺ cells that stained with the D^{b}/E1A tetramers was approximately 3%. In mice vaccinated with whole adenovirus, which induces potent E1A-specific immunity, comparable amounts of D^{b}/E1A tetramer-reactive CD8⁺ spleen cells were detected. These results indicate that the expansion of E1A-specific CD8⁺ T cells in mice that received the E1A/IFA vaccine in combination with the anti-CD40 mAb was substantial and equivalent to that found in virus vaccinated animals.

### Example 4: CD40-triggering strongly enhances the efficacy of peptide-based anti-cancer vaccines

Although the findings described above show that provision of help through CD40-triggering is sufficient to prevent CTL-tolerization after administration of a tolerogenic peptide-vaccine, they do not address the question whether the efficacy of anti-cancer vaccines that normally induce protective immunity, instead of tolerance, can be enhanced by activation through CD40. It was examined whether CD40-triggering *in vivo* is beneficial to the outcome of vaccination with an HPV16 E7-derived peptide. Vaccination with this peptide induces protective CTL-mediated immunity against a challenge with HPV16-transformed tumor cells. Moreover, this peptide can be used in a therapeutic setting when loaded on *in vitro* activated DC suggesting that the strength of the anti-tumor response is enhanced when presented by activated DC.

Mice receiving the E7-peptide in combination with CD40-triggering mounted a more potent CTL-response compared to mice treated with E7-peptide only (data not shown), indicating that CD40-triggering also enhances the efficacy of the HPV16 E7-peptide vaccine and confirming the findings with the E1A peptide described above. Moreover, mice treated 6 days after s.c. injection of CD40-negative HPV16 E6/E7 transformed tumor cells with the HPV16 E7-peptide alone (open squares) are able to slow down tumor growth, but eventually most animals succumb to the tumor (Figure, 8). When, however, HPV 16 E7-peptide vaccination was combined with injection of the anti-CD40 mAb, tumor growth was markedly reduced and 7 out of 10 mice rejected the tumor, whereas animals injected with a control peptide and the anti-CD40 mAb were unable to control outgrowth of the tumor. These results show that the effect of vaccination regiments can be markedly enhanced when immunization is combined with *in vivo* CD40-triggering. These data provide the basis for the development of extremely potent and novel anti-tumor vaccines for cancer patients.

### Example 5: In vivo triggering through 4-1BB provides a licence to kill to naïve CTL

In vivo CD40 triggering of APC through the administration of an agonistic anti-CD40 antibody (Ab) can replace the need for CD4⁺ T helper (Th) activity in cross-priming of Ad5-specific CTL. We injected Ad5E1-transformed mouse embryo cells (Ad5MEC) into syngeneic CD4⁺ Th cell-depleted C57BL/6 (B6) mice in combination with either anti-CD40 or anti-4-1BB Ab. These Ad5E1MEC were derived from TAP-deficient mice, and are therefore incapable of presenting the Ad5EI encoded-CTL epitopes in the context of their own MHC (20).

Consequently, priming of Ad5E1-specific CTL in this setting relies on cross-presentation of tumor antigens by host APC. The induction of Ad5EIB-specific CTL is CD4⁺ Th-dependent, as mice depleted for CD4⁺ T cells fail to mount an E1B-specific CTL response (Fig. 9b). Systemic administration of agonistic anti-CD40 Ab in these CD4-depleted animals restored E1B-specific cross priming (Fig. 9c). Interestingly, systemic administration of an agonistic anti-4-1BB Ab resulted in equally efficient priming of E1B-specific CTL in the absence of CD4⁺ T cell help (Fig. 9d). A different method of inducing antigen-specific CTL immunity against tumors is through vaccination with minimal peptide-epitopes in adjuvant (IFA). Notably, in case of the epitopes derived from AdSE1A and E1B such peptide-based vaccines induce CTL tolerance rather than priming, unless agonistic anti-CD40 Ab is co-administered (4). We, therefore, investigated whether also in this setting a trigger through 4-1BB could permit CTL priming by injecting B6 mice with the E1A-peptide vaccine in combination with anti 4-1BB Ab, anti-CD40 Ab or control antibody. Indeed, not only co-injection of anti-CD40, but also co-injection of anti-4-1BB converted the tolerogenic peptide vaccine into an immunogenic formulation (fig 1E-G). Similar results were obtained when E1A-specific CTL immunity was monitored by staining of splenocytes with H-2D^{d}/E1A peptide tetramers (not shown). Thus, in vivo triggering of 4-1BB can prevent the induction of peptide-specific tolerance, and instead result in the priming of a potent E1A-specific CTL response. Taken together, these data demonstrate that the 4-1BB signal efficiently provides CTL with a licence to kill, and as such can replace the need for CD40 triggering through Th cells or administration of agonistic anti-CD40 Ab.

### Example 6: Anti-4-1BB Ab does not induce DC activation

Current knowlegde of the expression patterns of CD40 and 4-1BB would argue that the signal provided by in vivo administration of anti-CD40 Ab is delivered to the APC, whereas that of anti-4-1BB Ab is delivered directly to the CTL. To exclude the possibility that the anti-4-1BB antibody, instead of directly stimulating antigen-specific T cells, would mediate its effect on CTL priming (Fig. 9) in an indirect fashion by activation of the antigen presenting DC, we investigated whether injection of the anti-4-1BB antibody activates DC in vivo. B6 mice were injected with anti-4-1BB, anti-CD40 or control Ab and the CD86 (B7.2) expression on CD11c⁺ DC isolated from the spleens of these mice was analysed. As expected, in vivo triggering of CD40 resulted in an enhanced expression of CD86, indicating in vivo activation of the DC. In contrast, after in vivo 4-1BB triggering or administration of control rat Ab the expression of CD86 on DC was not increased (Fig. 10a). The failure of the anti-4-1BB Ab to induce DC activation is in accordance with the fact that neither immature nor mature DC express detectable levels of 4-1BB, implicating that DC are not receptive for 4-1BB triggering (Fig. 10b). Therefore, anti-4-1BB antibody does not appear to affect the DC population, but rather mediates its stimulatory effect on CTL priming through direct stimulation of CD8⁺ T cells.

### Example 7: Costimulation through 4-1BB results in increased survival of antigen-stimulated CTL

To investigate in which manner agonistic anti-4-1BB Ab permits T helper independent priming of Ad5-specific CTL, we exploited adoptively transferred T cell populations from Ad5E1A-peptide specific T cell receptor (TCR) transgenic mice (21). Tracking of these T cells in vivo was performed in two distinct ways. First, TCR transgenic T cells were fluorescently labelled with the dye CFSE and transferred into normal B6 mice, after which these mice were vaccinated with the AdSE1A-peptide in IFA with or without anti-4-1BB Ab treatment. Interestingly, the resulting data revealed that proliferation of the TCR-transgenic T cells was equally triggered in mice that had received the E1A-peptide either with or without anti-4-1BB Ab (Fig. 11a). Therefore anti-4-1BB Ab do not enable CTL priming by increasing the initial activation or proliferative capacity of antigen-specific T cells.
In a second approach we transferred Ad5E1A-specific TCR transgenic T cells, which were from B6 Thy1.2 origin, into B6 Thy1.1 congenic mice. These mice were vaccinated with the Ad5E1A-peptide in IFA with or without anti-4-1BB Ab treatment. Figure 3b shows that at 11 days after vaccination, massive expansion of CD8⁺T cells from TCR-transgenic origin was observed in mice vaccinated with E1A-peptide in combination with anti-4-1BB Ab, but not in mice that received either E1A-peptide or anti-4-1BB Ab alone. Therefore, accumulation of E1A-specific CTL required the presence of the 4-1BB costimulatory signal. Furthermore, stimulation of CD8⁺ CTL immunity through 4-1BB was dependent on the presence of an antigen-specific trigger. Taken together, the data in figure 3 demonstrate that 4-1BB triggering is not essential for initial CTL activation, but promotes the survival of antigen-stimulated CTL, thereby permitting expansion of these CTL.

### Example 8: Involvement of 4-IBBL/4-1BB interactions in CTL priming

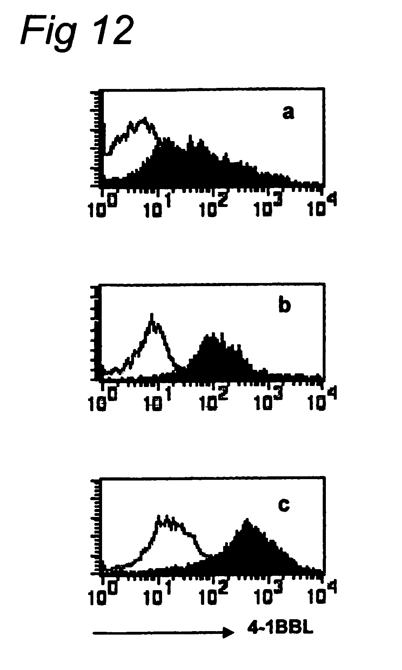

Because in vivo triggering of 4-1BB allows for CTL priming in the absence of the CD40-dependent DC activation signal (Fig. 9), this implies that antigen presentation by nonactivated DC in combination with an activating anti-4-1BB Ab suffices for efficient CTL priming. The need for co-administration of anti-4-1BB Ab also indicated that nonactivated DC were apparently not capable of stimulating CTL through 4-1BB. Therefore, we analyzed expression of the natural ligand for this receptor, 4-1BBL, on immature and mature DC. Non-activated DC express low levels of 4-1BBL (Fig. 12a), whereas the expression of this molecule is strongly increased upon activation in the presence of the CD40L-trimer- or Poly I:C (Fig. 12b and c, respectively). This induction of 4-1BBL was observed upon CD40 triggering of DC in vivo. The increase in 4-1BBL expression was paralleled by elevated levels of CD86 on the activated DC. This suggests that both costimulatory signals contribute to the capacity of mature DC, as opposed to immature DC, to induce CTL immunity.

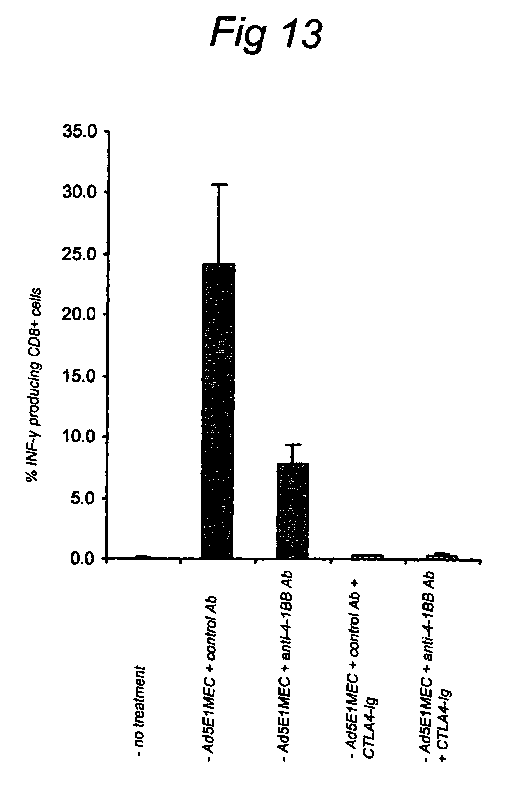

We performed tumor cell immunization experiments in normal, CD4⁺ Th cell proficient B6 mice, in which the Th-dependent DC maturation signal required for CTL cross-priming is intact. Blockade of the CD28 and 4-1BB signals was performed by administration of blocking anti-4-1BBL Ab or CTLA4-Ig respectively. B6 mice were immunized with Ad5E1MEC in combination with systemic doses of anti-4-1BBL Ab and/or CTLA4-Ig. Interestingly, in vivo blockade of anti-4-1BBL resulted in a marked reduction in the number of antigen-specific CTL induced by the tumor cell vaccine (Fig. 13). Blockade of CD28 costimulation completely inhibited CTL induction and, therefore, additional 4-1BBL blocking in combination with CTLA4-Ig had no extra effect. These data indicate that 4-1BBL/4-1BB interactions are important for the cross-priming of antigen-specific CTL, as blocking of the interaction between these molecules greatly diminishes the numbers of tumor-specific CTL that are sustained. However in the absence of the co-stimulatory signal through CD28, provided by properly activated DC, the signal provided by 4-1BBL is apparently not capable of enabling CTL priming and, as a result, induction of anti-tumor CTL immunity is completely abrogated.

### Example 9: The 4-1BB signal depends on CD28 costimulation

The in vivo blocking experiments suggested a dominance of CD28 costimulation over the 4-1BB signal. Therefore, we investigated whether in the absence of CD28 costimulation, administration of anti-4-1BB Ab would still provide CTL with a licence to kill. B6 mice were depleted for CD4⁺ T cells and immunised with Ad5E1MEC in combination with administration of anti-4-1BB Ab and/or CTLA4-Ig. Mice that received anti-4-1BB Ab mounted strong E1B-specific CTL responses, but mice that in addition received CTLA4-Ig failed to do so (Fig. 14). These data indicate that the positive effect of 4-1BB triggering on the induction of CTL immunity is dependent on the presence of an intact CD28 costimulatory signal. Furthermore, these data suggest that the basal levels of CD80/CD86 expressed by non-activated DC provide sufficient costimulation through CD28 to allow additional costimulation through the 4-1BB pathway.

In vitro studies have demonstrated that 4-1BB expression on naive T cells is absent and that TCR-triggering, through cross-linking by anti-CD3 Ab, results in a rapid increase in 4-1BB surface expression (8). As our data support the conclusion that costimulation through CD28 is prerequisite for 4-1BB signaling, we investigated whether CD28 triggering contributes to the up-regulation of 4-1BB on naive T cells. Therefore, naive spleen cells were stimulated in vitro with plate-bound anti-CD3 Ab and analyzed for 4-1BB expression 24h later (Fig. 16). It is clear that strong signals through the TCR (high concentrations of anti-CD3 Ab) are sufficient to induce 4-1BB expression within 24h. However, when T cells are stimulated with lower anti-CD3 concentrations, their capacity to upregulate 4-1BB is largely lost (Fig. 16). These lower anti-CD3 Ab concentrations provide a weaker TCR trigger that is more likely to resemble an in vivo signal of the kind provided by the tumor cell vaccine. Importantly, under these more physiological conditions, costimulation through the CD28 receptor restored the capacity of T cells to express high levels of 4-1BB. These findings are in accordance with the fact that blockade of the CD28 pathway in vivo abrogates costimulation through 4-1BB (Fig. 14 and 15). Furthermore, they strengthen the notion that CD28-costimulation of antigen-stimulated T cells is an important signal for 4-1BB upregulation on naive T cells, thereby making these cells susceptible for 4-1BB triggering.

### Example 10: Enabling of CTL priming by a tumor cell or peptide-based vaccine through administration of a synergistic combination of anti-CD40 and anti-4-1BB Ab

Because the anti-CD40 Ab and anti-4-1BB Ab enabled CTL priming by the E1A peptide vaccine in a similar fashion, while 4-1BB triggering was dependent on CD28-costimulation, we investigated whether combination of both Ab would result in even greater induction of CTL immunity. Our data revealed that combination of the E1A peptide vaccine with both Ab indeed has strong synergistic effects on induction of the CTL response. In mice that received both Ab the absolute as well as relative numbers of tetramer-positive CD8⁺ T cells were increased at least 5-10 fold as compared to mice that received only one of the Ab (Fig. 17).

### Example 11: Induction of therapeutic anti-tumor immunity through administration of agonistic anti-CD40 Ab to tumor-bearing subjects.

Since in vivo activation of APC through injection of anti-CD40 Ab enables cross-priming of tumor-specific CTL in tumor cell-immunized CD4⁺ T cell depleted mice, this procedure could also promote the induction of anti-tumor immunity in tumor-bearing animals. Initiation of anti-tumor CTL responses in tumor-bearing mice is most likely hampered by the lack of antigen presentation by properly activated APC. This could be due to insufficient APC recruitment to the tumor site and/or the failure of tumor antigen-loaded APC to become activated and redistributed to the draining lymph nodes, the site where encounter with - and priming of - naive T cells takes place. Especially lack of inflammatory 'danger' signals, lack of tumor-specific T cell help and the secretion of immunomodulatory cytokines by the tumor could result in the failure of APC to cross-prime anti-tumor CTL responses (22).

In view of these considerations, we injected immunocompetent B6 mice, bearing (subcutaneous) tumors of Ad5-transformed cells, with anti-CD40 Ab. Whereas tumors grew progressively in control animals, anti-CD40 treated mice were capable of rejecting their tumors (Fig. 18). Interestingly, in control animals we only detected low numbers of E1A-specific CTL in the draining lymph nodes, whereas anti-CD40 treated mice clearly exhibited elevated numbers of E1A-specific CTL in both draining and co-lateral lymph nodes as well as in peripheral blood (Fig. 19a-c).

Importantly, the localization of antigen presentation is similar in anti-CD40 treated and control mice, in that in both cases this is only detected in the tumor-draining lymph node, not in the co-lateral lymph node (Figure 20). As described above, due to a lack of inflammatory 'danger' signals the APC apparently fail to cross-prime anti-tumor CTL responses in the control animals. In contrast, in the anti-CD40 Ab-treated mice the CD40-signal empowers these APC to effectively prime AdSE1A-specific CTL immunity and, as a result, these CTL are capable of leaving the draining lymph node and gain access to other peripheral lymphoid organs as well as to the tumor. This latter point is confirmed by the fact that A5E1A-specific CTL are primarily detected in the tumors of anti-CD40 Ab-treated mice (Figure 21). The notion that anti-CD40 treatment results in effective systemic CTL immunity is further supported by the fact that local in vivo triggering through CD40, by intra-tumoral injection of the anti-CD40 Ab into the tumor on one flank, also results in rejection of the non-treated tumor on the other flank (Fig. 22a-c). This experiment also demonstrates that effective anti-tumor therapy can be achieved both after systemic and local administration of the anti-CD40 Ab.

Two additional experiments provide further insight into the mechanisms through which anti-CD40 Ab injection results in tumor rejection. First, the role of these CTL in tumor eradication was confirmed by the fact that anti-tumor therapy with anti-CD40 Ab is not effective in mice that are depleted of their CD8⁺ T cells, while still being effective in mice that are depleted of their CD4⁺ T cells (Fig. 23). Secondly, it should be noted that others have found anti-CD40 Ab to inhibit growth of CD40-expressing tumors through direct induction of tumor cell apoptosis (23-25). Importantly, our tumor cells lack CD40 expression (Fig. 24). Therefore, the therapeutic effect of the anti-CD40 Ab in this setting acts through the induction of a tumoricidal CTL response.

### Example 12: Induction of therapeutic anti-tumor immunity through administration of a synergistic combination of anti-CD40 and anti-4-1BB Ab to tumor-bearing subjects.

Our data show that administration of agonistic anti-CD40 Ab, in addition to enabling CTL priming against tumor cell- and peptide-based vaccines, can also empower the immune system to raise therapeutic CTL immunity against pre-existing tumors. Because the anti-CD40 and 4-1BB Ab are similarly capable of stimulating CTL immunity, and act synergistically in stimulating peptide vaccine-induced responses (see above), we expect a very powerful therapeutic capacity this potent Ab-combination concerning the induction of CTL immunity in tumor-bearing mice.

The foregoing description, terms, expressions and examples are exemplary only and not limiting. The invention includes all equivalents of the foregoing embodiments, both known and unknown. The invention is limited only by the claims which follow and not by any statement in any other portion of this document or in any other source.

### References:

1. Schoenberger, S. P., R. E. Toes, E. I. van der Voort, R. Offringa, C. J. Melief. 1998. T-cell help for cytotoxic T lymphocytes is mediated by CD40-CD40L interactions. Nature. 393: 480-3.
2. Ridge, J. P., F. Di Rosa, P. Matzinger. 1998. A conditioned dendritic cell can be a temporal bridge between a CD4+ T-helper and a T-killer cell. Nature. 393: 474-8.
3. Bennett, S. R., F. R. Carbone, F. Karamalis, R. A. Flavell, J. F. Miller, W. R. Heath. 1998. Help for cytotoxic-T-cell responses is mediated by CD40 signalling. Nature. 393: 478-80.
4. Diehl, L., A. T. den Boer, S. P. Schoenberger, E. I. van der Voort, T. N. Schumacher, C. J. Melief, R. Offringa, R. E. Toes. 1999. CD40 activation in vivo overcomes peptide-induced peripheral cytotoxic T-lymphocyte tolerance and augments anti-tumor vaccine efficacy. Nat Med. 5: 774-9.
5. Banchereau, J., F. Briere, C. Caux, J. Davoust, S. Lebecque, Y. J. Liu, B. Pulendran, K. Palucka. 2000. Immunobiology of dendritic cells. Annu Rev Immunol. 18: 767-811
6. Lenschow, D. J., T. L. Walunas, J. A. Bluestone. 1996. CD28B7 system of T cell costimulation. Annu Rev Immunol. 14: 233-58
7. Chambers, C. A., J. P. Allison. 1999. Costimulatory regulation of T cell function. Curr Opin Cell Biol. 11: 203-10.
8. Hurtado, J. C., Y. J. Kim, B. S. Kwon. 1997. Signals through 4-1BB are costimulatory to previously activated splenic T cells and inhibit activation-induced cell death. J Immunol. 158: 2600-9.
9. Takahashi, C., R. S. Mittler, A. T. Vella. 1999. Cutting edge: 4-1BB is a bona fide CD8 T cell survival signal. J Immunol. 162: 5037-40.
10. Goodwin, R. G., W. S. Din, T. Davis-Smith, D. M. Anderson, S. D. Gimpel, T. A. Sato, C. R. Maliszewski, C. I. Brannan, N. G. Copeland, N. A. Jenkins, et al. 1993. Molecular cloning of a ligand for the inducible T cell gene 4-1BB: a member of an emerging family of cytokines with homology to tumor necrosis factor. Eur J Immunol. 23: 2631-41.
11. Alderson, M. R., C. A. Smith, T. W. Tough, T. Davis-Smith, R. J. Armitage, B. Falk, E. Roux, E. Baker, G. R. Sutherland, W. S. Din. 1994. Molecular and biological characterization of human 4-1BB and its ligand. Eur J Immunol. 24: 2219-27.
12. DeBenedette, M. A., A. Shahinian, T. W. Mak, T. H. Watts. 1997. Costimulation of CD28- T lymphocytes by 4-1BB ligand. J Immunol. 158: 551-9.
13. Shuford, W. W., K. Klussman, D. D. Tritchler, D. T. Loo, J. Chalupny, A. W. Siadak, T. J. Brown, J. Emswiler, H. Raecho, C. P. Larsen, T. C. Pearson, J. A. Ledbetter, A. Aruffo, R. S. Mittler. 1997. 4-1BB costimulatory signals preferentially induce CD8+ T cell proliferation and lead to the amplification in vivo of cytotoxic T cell responses. J Exp Med. 186: 47-55.
14. DeBenedette, M. A., N. R. Chu, K. E. Pollok, J. Hurtado, W. F. Wade, B. S. Kwon, T. H. Watts. 1995. Role of 4-1BB ligand in costimulation of T lymphocyte growth and its upregulation on M12 B lymphomas by cAMP. J Exp Med. 181: 985-92.
15. Hurtado, J. C., S. H. Kim, K. E. Pollok, Z. H. Lee, B. S. Kwon. 1995. Potential role of 4-1BB in T cell activation. Comparison with the costimulatory molecule CD28. J Immunol. 155: 3360-7.
16. DeBenedette, M. A., T. Wen, M. F. Bachmann, P. S. Ohashi, B. H. Barber, K. L. Stocking, J. J. Peschon, T. H. Watts. 1999. Analysis of 4-1BB ligand (4-1BBL)-deficient mice and of mice lacking both 4-1BBL and CD28 reveals a role for 4-1BBL in skin allograft rejection and in the cytotoxic T cell response to influenza virus. J Immunol. 163: 4833-41.
17. Tan, J. T., J. K. Whitmire, R. Ahmed, T. C. Pearson, C. P. Larsen. 1999. 4-1BB ligand, a member of the TNF family, is important for the generation of antiviral CD8 T cell responses. J Immunol. 163: 4859-68.
18. Tan, J. T., J. K. Whitmire, K. Murali-Krishna, R. Ahmed, J. D. Altman, R. S. Mittler, A. Sette, T. C. Pearson, C. P. Larsen. 2000. 4-1BB costimulation is required for protective anti-viral immunity after peptide vaccination. J Immunol. 164: 2320-5.
19. Melero, I., W. W. Shuford, S. A. Newby, A. Aruffo, J. A. Ledbetter, K. E. Hellstrom, R. S. Mittler, L. Chen. 1997. Monoclonal antibodies against the 4-1BB T-cell activation molecule eradicate established tumors. Nat Med. 3: 682-5.
20. Schoenberger, S. P., E. I. van der Voort, G. M. Krietemeijer, R. Offringa, C. J. Melief, R. E. Toes. 1998. Cross-priming of CTL responses in vivo does not require antigenic peptides in the endoplasmic reticulum of immunizing cells. J Immunol. 161: 3808-12.
21. Toes, R. E., R. Offringa, R. J. Blom, C. J. Melief, W. M. Kast. 1996. Peptide vaccination can lead to enhanced tumor growth through specific T-cell tolerance induction. Proc Natl Acad Sci USA. 93: 7855-60.
22. Melief, C. J., R. E. Toes, J. P. Medema, S. H. van der Burg, F. Ossendorp, R. Offringa. 2000. Strategies for immunotherapy of cancer. Adv Immunol. 75: 235-82
23. Hess, S., H. Engelmann. 1996. A novel function of CD40: induction of cell death in transformed cells. J Exp Med. 183: 159-67.
24. Hirano, A., D. L. Longo, D. D. Taub, D. K. Ferris, L. S. Young, A. G. Eliopoulos, A. Agathanggelou, N. Cullen, J. Macartney, W. C. Fanslow, W. J. Murphy. 1999. Inhibition of human breast carcinoma growth by a soluble recombinant human CD40 ligand. Blood. 93: 2999-3007.
25. Young, L. S., A. G. Eliopoulos, N. J. Gallagher, C. W. Dawson. 1998. CD40 and epithelial cells: across the great divide. Immunol Today. 19: 502-6.

## Claims

1. The use of an agonistic anti-CD40 antibody, or a fragment thereof which stimulates the CD40 receptor, in the manufacture of a medicament for the treatment of a tumour or infectious agent by induction of systemic T cell immunity against an antigen of the tumour or infectious agent, whereby the treatment does not comprise immunisation with an antigen of the tumour or infectious agent.

2. The use according to claim 1, wherein the infected or tumour cells do not express the CD40 receptor.

3. The use according to claim 1 or 2 wherein the CD40 receptor targeted by the agonistic anti-CD40 antibody is expressed on the dendritic cells of the treated subject.

4. The use according to any of the preceding claims, wherein the induction of the systemic T cell immunity is a cytotoxic T cell response.

5. The use according to any of the preceding claims, wherein the agonistic anti-CD40 antibody or fragment thereof is human, humanised, chimeric or deimmunised.

6. The use according to any of the preceding claims, wherein the fragment is a V_{H}, V_{L}, Fv, Fd, Fab, (Fab)₂ or scFv fragment of a human antibody.

7. The use according to any of the preceding claims, wherein the medicament is for injection or oral administration.

8. The use according to any of the preceding claims, wherein the injection is an intra-tumoral injection.

9. The use according to any of the preceding claims, wherein the antigen is a tumour-specific antigen.

10. The use according to any of the preceding claims, wherein the antigen is an antigen of human papilloma virus (HPV) or adenovirus.

## Patentansprüche

1. Verwendung eines den CD40-Rezeptor stimulierenden, agonistischen anti-CD40-Antikörpers oder eines dessen Fragmente zur Herstellung eines Arzneimittels zur Behandlung eines Tumors oder eines Infektionserregers durch Induktion systemischer T-Zellen-Immunität gegenüber einem Antigen des Tumors bzw. Infektionserregers, **dadurch gekennzeichnet, dass** die Behandlung keine Immunisierung mit einem Antigen des Tumors bzw. Infektionserregers umfasst.

2. Verwendung gemäß Anspruch 1, **dadurch gekennzeichnet, dass** die infizierten Zellen bzw. die Tumorzellen den CD40-Rezeptor nicht exprimieren.

3. Verwendung gemäß Anspruch 1 oder 2, **dadurch gekennzeichnet dass** der CD40-Rezeptor, auf den der agonistische anti-CD40-Antikörper abgestimmt ist, auf den dendritischen Zellen des zu behandelnden Versuchsobjekts exprimiert wird.

4. Verwendung gemäß einem der vorstehenden Ansprüche, **dadurch gekennzeichnet, dass** die Induktion einer systemischen T-Zellen-Immunität eine Antwort der zytotoxischen T-Zellen darstellt.

5. Verwendung gemäß einem der vorstehenden Ansprüche, **dadurch gekennzeichnet, dass** der agonistische CD40-Antikörper oder eines dessen Fragmente human, humanisiert, chimärisch oder deimmunisiert ist.

6. Verwendung gemäß einem der vorstehenden Ansprüche, **dadurch gekennzeichnet, dass** das Fragment ein V_{H}-, V_{L}-, FV-, Fd-, Fab-, (Fab)₂- oder scFv-Fragment eines humanen Antikörpers darstellt.

7. Verwendung gemäß einem der vorstehenden Ansprüche, **dadurch gekennzeichnet, dass** das Arzneimittel zur Verabreichung per Injektion oder orale Gabe bestimmt ist.

8. Verwendung gemäß einem der vorstehenden Ansprüche, **dadurch gekennzeichnet, dass** die Injektion eine intratumorale Injektion ist.

9. Verwendung gemäß einem der vorstehenden Ansprüche, **dadurch gekennzeichnet, dass** das Antigen ein tumorspezifisches Antigen ist.

10. Verwendung gemäß einem der vorstehenden Ansprüche, **dadurch gekennzeichnet, dass** das Antigen ein Antigen des humanen Papillomavirus (HPV) oder Adenovirus ist.

## Revendications

1. Utilisation d'un anticorps anti-CD40 agoniste ou d'un fragment de celui-ci, qui stimule le récepteur CD40, pour la fabrication d'un médicament destiné au traitement d'une tumeur ou d'un agent infectieux, par induction de l'immunité cellulaire T systémique contre un antigène de la tumeur ou de l'agent infectieux, lequel traitement ne comprend pas l'immunisation avec un antigène de la tumeur ou de l'agent infectieux.

2. Utilisation selon la revendication 1, dans laquelle les cellules infectées ou tumorales n'expriment pas le récepteur CD40.

3. Utilisation selon la revendication 1 ou 2, dans laquelle le récepteur CD40 ciblé par l'anticorps anti-CD40 agoniste est exprimé sur les cellules dendritiques du sujet traité.

4. Utilisation selon l'une quelconque des revendications précédentes, dans laquelle l'induction de l'immunité cellulaire T systémique est une réponse des lymphocytes T cytotoxiques.

5. Utilisation selon l'une quelconque des revendications précédentes, dans laquelle l'anticorps anti-CD40 agoniste ou un fragment de celui-ci, est humain, humanisé, chimérique ou désimmunisé.

6. Utilisation selon l'une quelconque des revendications précédentes, dans laquelle le fragment est un fragment V_{H}, V_{L}, Fv, Fd, Fab, (Fab)₂ ou scFv d'un anticorps humain.

7. Utilisation selon l'une quelconque des revendications précédentes, dans laquelle le médicament est destiné à une administration par injection ou par voie orale.

8. Utilisation selon l'une quelconque des revendications précédentes, dans laquelle l'injection est une injection intra-tumorale.

9. Utilisation selon l'une quelconque des revendications précédentes, dans laquelle l'antigène est un antigène spécifique de la tumeur.

10. Utilisation selon l'une quelconque des revendications précédentes, dans laquelle l'antigène est un antigène du papillomavirus humain (HPV) ou d'un adénovirus.
